# EUROPEAN PATENT APPLICATION

(11) **EP 4 282 873 A1**
(43) Date of publication of application: **29.11.2023**
(21) Application number: 22742673.1
(22) Date of filing: 21.01.2022
(51) Int. Cl.: C07K 7/64, C12N 15/11, C40B 50/06

(54) **METHOD FOR SCREENING DIMERIZED CYCLIC PEPTIDES**

(30) Priority: 22.01.2021 JP 2021008420
(71) Applicant: AJINOMOTO CO., INC., Chuo-ku Tokyo 104-8315 (JP)
(72) Inventor: ITO, Kenichiro, Kawasaki-shi, Kanagawa 210-8681 (JP); MIYAIRI, Kyohei, Kawasaki-shi, Kanagawa 210-8681 (JP)
(74) Representative: Hoffmann Eitle
(86) International application number: PCT/JP2022/002117
(87) International publication number: WO 2022/158554

(57) **Abstract**

Provided is a quick and simple method for screening dimerized cyclic peptides, a library therefor, and molecules including cyclic peptides that can be used in the method and the library. For example, the method for screening dimerized cyclic peptides includes (1) a step in which, in a cell-free expression system, a cyclic peptide, a dimerization domain including a cysteine residue and a leucine zipper region, and at least one polypeptide including a peptide linker existing between the cyclic peptide and the dimerization domain are synthesized, and a dimer of the polypeptide is prepared, and (2) a step in which activity based on binding between the dimer and a target molecule is measured.

## Description

### Technical Field

The present invention relates to a method for screening a dimerized cyclic peptide, a library therefor, a molecule including a cyclic peptide that can be used therefor, and the like.

### Background Art

In recent years, there has been a surge in the research and application of biopharmaceuticals and other antibody drugs, regenerative medicine, and nucleic acid drugs as pharmaceutical modalities. Furthermore, medium-sized molecules, particularly peptides, have emerged as a promising new pharmaceutical modality. Notably, several peptides with distinctive shapes (special peptides), including cyclic peptides, have been developed and hold potential for use in pharmaceuticals.

Compared to linear peptides, cyclic peptides offer superior binding affinity, target selectivity, and resistance to degradation. Thus, the use of cyclic peptides in pharmaceuticals and other fields is highly promising.

Some peptides form dimers to exhibit biological activity or enhanced biological activity. For instance, growth factors, which are biomolecules critical for cell differentiation, are an example thereof. In recent years, research has been conducted to develop alternative molecules that enhance the production cost and storage stability of these molecules that form dimers to exhibit biological activity, and the application of cyclic peptides is currently under consideration. Consequently, a rapid and uncomplicated method for screening dimerized cyclic peptides is eagerly anticipated.

The conventional approach to obtain peptide dimers by dimerizing cyclic peptides involves synthesizing a molecule by linking two cyclic peptides using a linker. However, in a biosynthetic system, to synthesize such a molecular format, the sequences of the two locations that correspond to the cyclic peptides in the template gene must be altered, and this necessitates complex procedures such as genetic recombination at multiple locations and whole gene synthesis. Consequently, this makes rapid and uncomplicated synthesis unattainable, and it cannot be utilized for rapid and uncomplicated screening of dimerized cyclic peptides.

Meanwhile, there is a known example of using Escherichia coli to express a molecule in which a peptide is linked to a leucine zipper region to obtain a peptide dimer through non-covalent interaction at the leucine zipper (International Publication No. WO2000/018921). However, International Publication No. WO2000/018921 intended to provide an alternative molecule to bFGF, not to screen peptide dimers, and does not describe any screening of peptide dimers.

### Summary of Invention

As described above, a rapid and uncomplicated method for screening dimerized cyclic peptides is desired.

An aspect of the present invention aims to provide a rapid and uncomplicated method for screening dimerized cyclic peptides, or a library therefor.

In addition, another aspect of the present invention aims to provide a rapid and uncomplicated method for screening dimerized cyclic peptides, or a molecule including a cyclic peptide that can be used in a library therefor.

The present invention may include, for example, the following aspects.
[1] A method for screening dimerized cyclic peptides, including steps of:
   (1) synthesizing at least one polypeptide including
      a cyclic peptide,
      a dimerization domain containing a cysteine residue and a leucine zipper region, and
      a peptide linker present between the cyclic peptide and the dimerization domain
      in a cell-free expression system to prepare a dimer of the polypeptide; and
   (2) measuring activity based on binding of the dimer to a target molecule.
[2] A method for screening dimerized cyclic peptides, including steps of:
   (1) synthesizing at least one polypeptide including
      a cyclic peptide,
      a dimerization domain containing a cysteine residue and a leucine zipper region, and
      a peptide linker present between the cyclic peptide and the dimerization domain.
      obtained by synthesizing the polypeptide in a cell-free expression system; and
   (2) measuring activity based on binding of the dimer to a target molecule.
[3] The method according to [1] or [2], wherein in step (1), the at least one polypeptide includes a plurality of different polypeptides expected to have binding affinity with the target molecule, and step (2) further includes measuring the activity of dimers of the plurality of different polypeptides based on binding to the target molecule.
[4] The method according to any one of [1] to [3], wherein step (1) further includes creating a library including a plurality of the at least one polypeptide or the dimer, or providing a library including a plurality of the at least one polypeptide or the dimer, and
   wherein the plurality of polypeptides include different cyclic peptides.
[5] A method for creating a library including a plurality of polypeptides or dimers of the plurality of polypeptide, including:
   synthesizing the plurality of polypeptides in a cell-free expression system, wherein
   the plurality of polypeptides each include
      a cyclic peptide,
      a dimerization domain containing a cysteine residue and a leucine zipper region, and
      a peptide linker present between the cyclic peptide and the dimerization domain, and
   the plurality of polypeptides include different cyclic peptides.
[6] A library for screening dimerized cyclic peptides, including:
   a plurality of polypeptides or dimers of the plurality of polypeptides; or
   a plurality of nucleic acids encoding the plurality of polypeptides, wherein
   the plurality of polypeptides each include
      a cyclic peptide,
      a dimerization domain containing a cysteine residue and a leucine zipper region, and
      a peptide linker present between the cyclic peptide and the dimerization domain, and
   the plurality of polypeptides include different cyclic peptides.
[7] The library according to [6], including 8 or more kinds of polypeptides or dimers of the 8 or more kinds of polypeptides, or 8 or more kinds of nucleic acids encoding the 8 or more kinds of polypeptides.
[8] A method for screening dimerized cyclic peptides using the library according to [6] or [7], including measuring activity based on binding of dimers of a plurality of polypeptides to a target molecule.
[9] A method for screening dimerized cyclic peptides, including steps of:
   (1) obtaining one or a plurality of polypeptides having binding affinity with a target molecule from a library including a plurality of displayed polypeptides, wherein the plurality of displayed polypeptides each contain
      a cyclic peptide,
      a dimerization domain containing a cysteine residue and a leucine zipper region, and
      a peptide linker present between the cyclic peptide and the dimerization domain, and
      the plurality of displayed polypeptides include different cyclic peptides,
   (2) recovering a nucleic acid associated with the polypeptides obtained in the step (1),
   (3) amplifying the nucleic acid recovered in the step (2),
   (4) synthesizing a polypeptide including
      a cyclic peptide,
      a dimerization domain containing a cysteine residue and a leucine zipper region, and
      a peptide linker present between the cyclic peptide and the dimerization domain
      in a cell-free expression system using the nucleic acid amplified in the step (3) to prepare a dimer of the polypeptide, and
   (5) measuring activity based on binding of the dimer to a target molecule.
[10] The method according to [9], wherein the step (1) includes creating a further library including a plurality of displayed polypeptides based on the obtained plurality of polypeptides, and repeating obtaining from the further library one or more polypeptides having binding affinity with the target molecule.
[11] A library for screening dimerized cyclic peptides, including:
   a plurality of displayed polypeptides; or
   a plurality of nucleic acids for expressing the plurality of displayed polypeptides, wherein
   the plurality of displayed polypeptides each include
      a cyclic peptide,
      a dimerization domain containing a cysteine residue and a leucine zipper region, and
      a peptide linker present between the cyclic peptide and the dimerization domain, and
   the plurality of displayed polypeptides include different cyclic peptides.
[12] The library according to [11], including 10⁵ or more kinds of displayed polypeptides or 10⁵ or more kinds of nucleic acids for expressing the 10⁵ or more kinds of displayed polypeptides.
[13] A method for obtaining dimerized cyclic peptides having activity based on binding to a target molecule by measuring the activity based on the binding to the target molecule by the method according to any one of [1] to [4] and [8] to [10].
[14] A method for preparing dimerized cyclic peptides, including:
   synthesizing a polypeptide including
      a cyclic peptide,
      a dimerization domain containing a cysteine residue and a leucine zipper region, and
      a peptide linker present between the cyclic peptide and the dimerization domain
   in a cell-free expression system to prepare a dimer of the polypeptide.
[15] A polypeptide including:
   a cyclic peptide,
   a dimerization domain containing a cysteine residue and a leucine zipper region, and
   a peptide linker present between the cyclic peptide and the dimerization domain.
[16] The polypeptide according to [15], wherein the dimerization domain has one cysteine residue.
[17] The polypeptide according to [15] or [16], wherein the peptide linker is composed of one or more amino acid residues selected from alanine (A), proline (P), and serine (S).
[18] A nucleic acid encoding the polypeptide according to any one of [15] to [17].

An embodiment of the present invention makes it possible to screen dimerized cyclic peptides having activity based on binding to a target molecule in a rapid and uncomplicated manner compared to conventional techniques.

An embodiment of the present invention makes it possible to provide highly efficient screening (high-throughput screening) for dimerized cyclic peptides having activity based on binding to a target molecule compared to conventional techniques.

An embodiment of the present invention makes it possible to provide low-cost screening for dimerized cyclic peptides having activity based on binding to a target molecule compared to conventional techniques.

An embodiment of the present invention makes it possible to provide screening for dimerized cyclic peptides having activity based on binding to a target molecule, which can be seamlessly assessed from acquiring displayed candidate polypeptides, which is not feasible with conventional techniques.

### Brief Description of Drawings

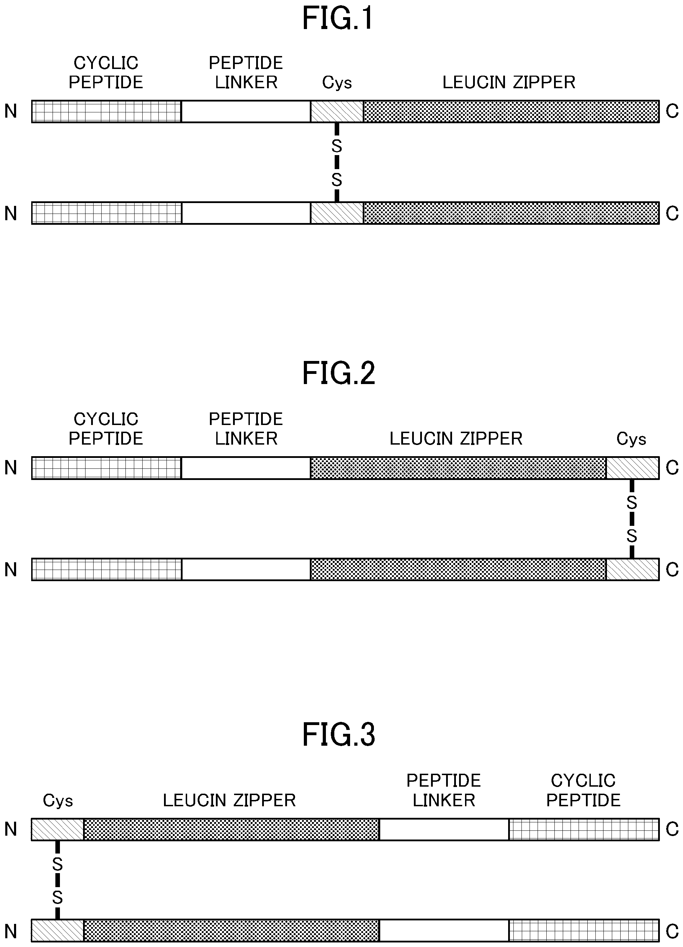

Fig. 1 is a schematic diagram of a dimerized cyclic peptide. The structure "cyclic peptide" - "peptide linker" - "cysteine residue" - "leucine zipper" is shown.
Fig. 2 is a schematic diagram of a dimerized cyclic peptide. The structure "cyclic peptide" - "peptide linker" - "leucine zipper" - "cysteine residue" is shown.
Fig. 3 is a schematic diagram of a dimerized cyclic peptide. The structure "cysteine residue" - "leucine zipper" - "peptide linker" - "cyclic peptide" is shown.

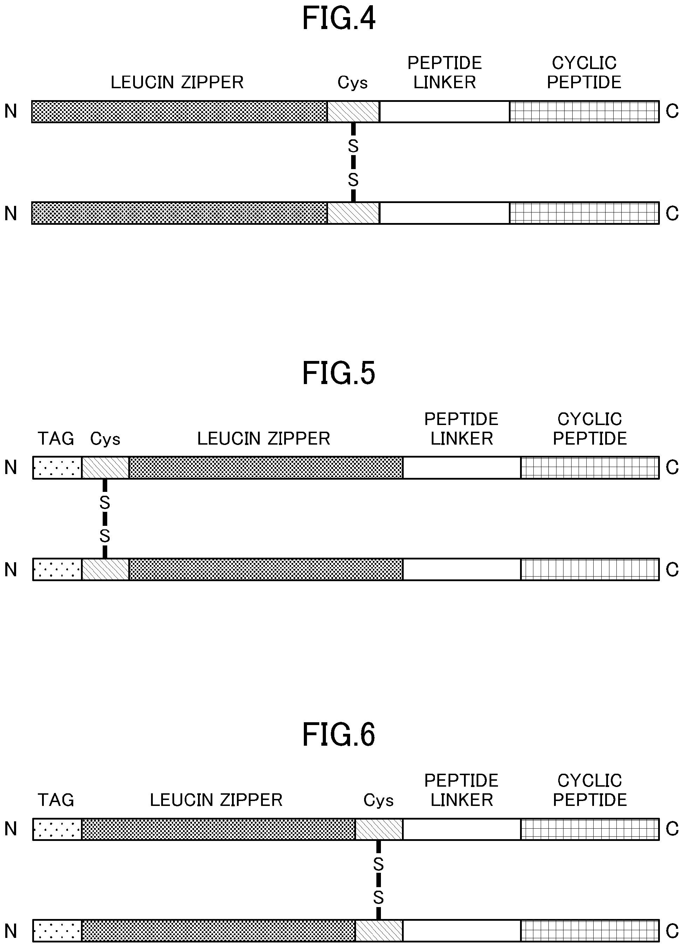

Fig. 4 is a schematic diagram of a dimerized cyclic peptide. The structure "leucine zipper" - "cysteine residue" - "peptide linker" - "cyclic peptide" is shown.
Fig. 5 is a schematic diagram of a dimerized cyclic peptide. The structure "tag sequence" - "cysteine residue" - "leucine zipper" - "peptide linker" - "cyclic peptide" is shown.
Fig. 6 is a schematic diagram of a dimerized cyclic peptide. The structure "tag sequence" - "leucine zipper" - "cysteine residue" - "peptide linker" - "cyclic peptide" is shown.

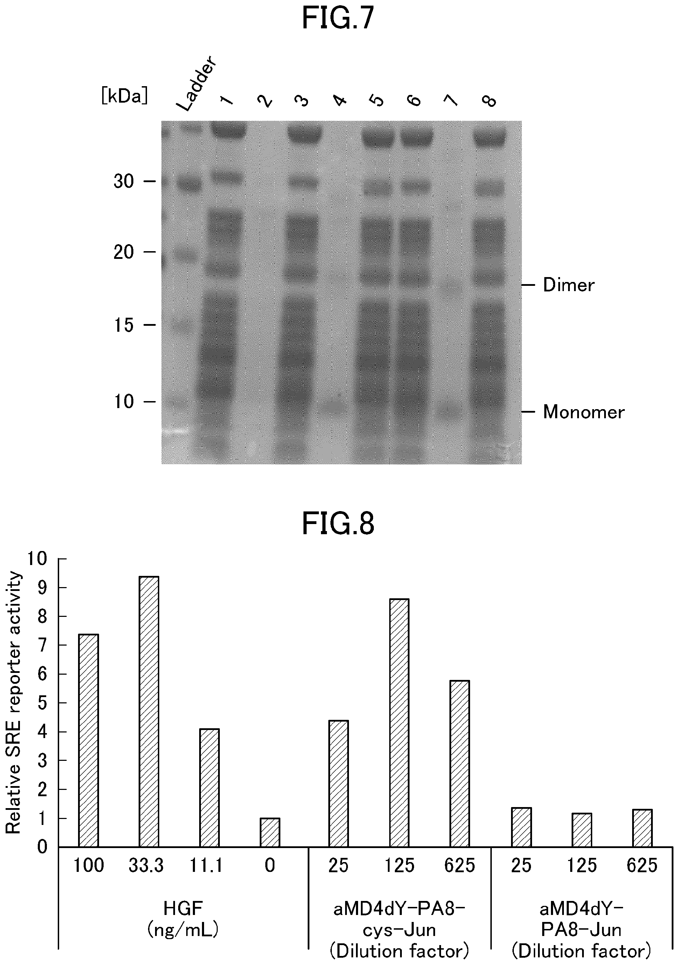

Fig. 7 is the result of SDS-polyacrylamide electrophoresis showing synthesis of dimerized cyclic peptides by a cell-free expression system. Ladder is a marker, and Lane 1 is a negative control (cell-free translation solution without addition of expression cDNA), Lane 2 is a fraction purified from the negative control using Dynabeads His-Tag Isolation & Pulldown (Thermo Fisher Scientific), Lane 3 is a cell-free translation solution of N11-Cys-Jun-PA8-aMD4dY, Lane 4 is a fraction purified from the cell-free translation solution of N11-Cys-Jun-PA8-aMD4dY using Dynabeads His-Tag Isolation & Pulldown, Lane 5 is a residual fraction thereof, Lane 6 is N11-Cys-GCN4-PA8-aMD4dY, Lane 7 is the fraction purified from the cell-free translation solution of N11-Cys-GCN4-PA8-aMD4dY using Dynabeads His-Tag Isolation & Pulldown, and Lane 8 is a residual fraction thereof. Monomer refers to the protein band putative to the cyclic peptide (aMD4dY)-leucine zipper fusion, and Dimer refers to the putative disulfide dimer thereof.
Fig. 8 is a graph showing activity evaluation of dimerized cyclic peptides (aMD4dY-PA8-cys-Jun and aMD4dY-PA8-Jun) synthesized with HGF. The vertical axis represents relative SRE receptor activity, and the horizontal axis represents the concentration for HGF, and for the synthesized dimerized cyclic peptides the dilution factor of the reaction solution of the cell-free expression system with respect to the medium subjected to activity evaluation.

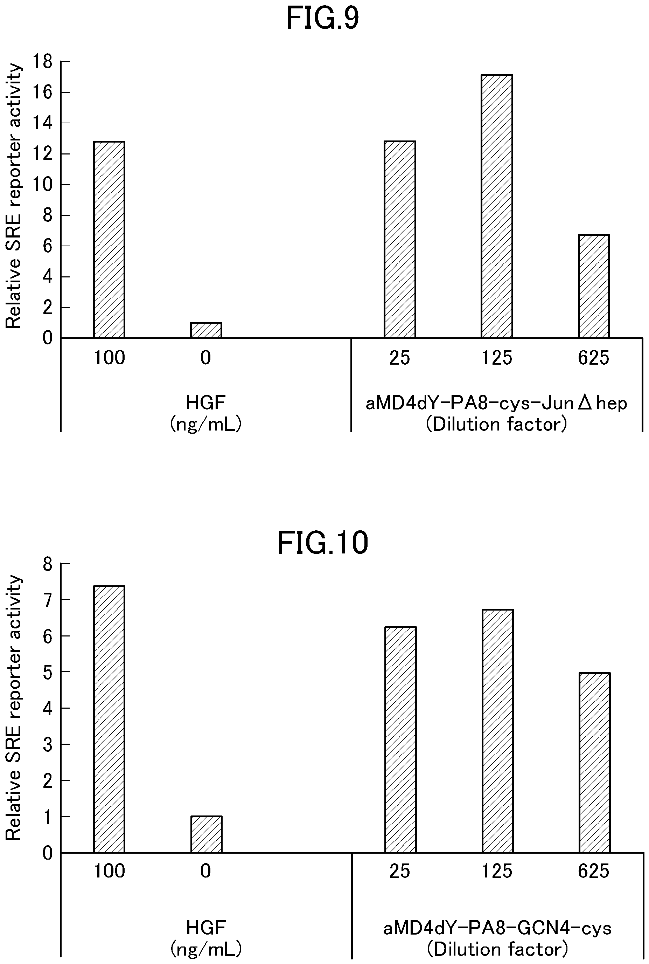

Fig. 9 is a graph showing activity evaluation of a dimerized cyclic peptide (aMD4dY-PA8-cys-JunΔhep) synthesized with HGF. The vertical axis represents relative SRE receptor activity, and the horizontal axis represents the concentration for HGF, and for the synthesized dimerized cyclic peptide the dilution factor of the reaction solution of the cell-free expression system with respect to the medium subjected to activity evaluation.
Fig. 10 is a graph showing activity evaluation of a dimerized cyclic peptide (aMD4dY-PA8-GCN4-cys) synthesized with HGF. The vertical axis represents relative SRE receptor activity, and the horizontal axis represents the concentration for HGF, and for the synthesized dimerized cyclic peptide the dilution factor of the reaction solution of the cell-free expression system with respect to the medium subjected to activity evaluation.

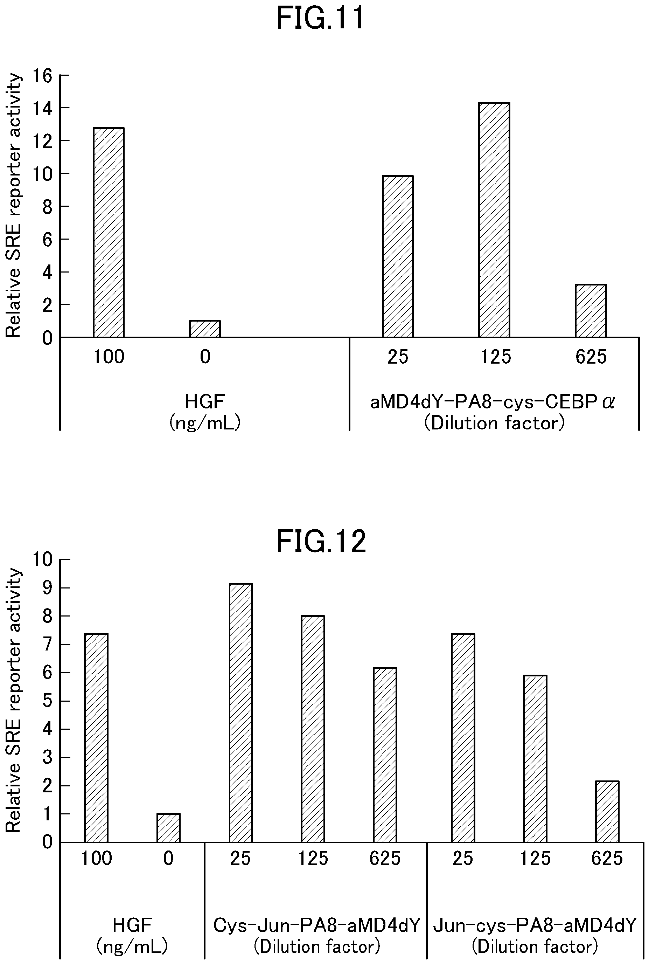

Fig. 11 is a graph showing activity evaluation of a dimerized cyclic peptide (aMD4dY-PA8-cys-CEBPα) synthesized with HGF. The vertical axis represents relative SRE receptor activity, and the horizontal axis represents the concentration for HGF, and for the synthesized dimerized cyclic peptide the dilution factor of the reaction solution of the cell-free expression system with respect to the medium subjected to activity evaluation.
Fig. 12 is a graph showing activity evaluation of dimerized cyclic peptides (cys-Jun-PA8-aMD4dY and Jun-cys-PA8-aMD4dY) synthesized with HGF. The vertical axis represents relative SRE receptor activity, and the horizontal axis represents the concentration for HGF, and for the synthesized dimerized cyclic peptides the dilution factor of the reaction solution of the cell-free expression system with respect to the medium subjected to activity evaluation.

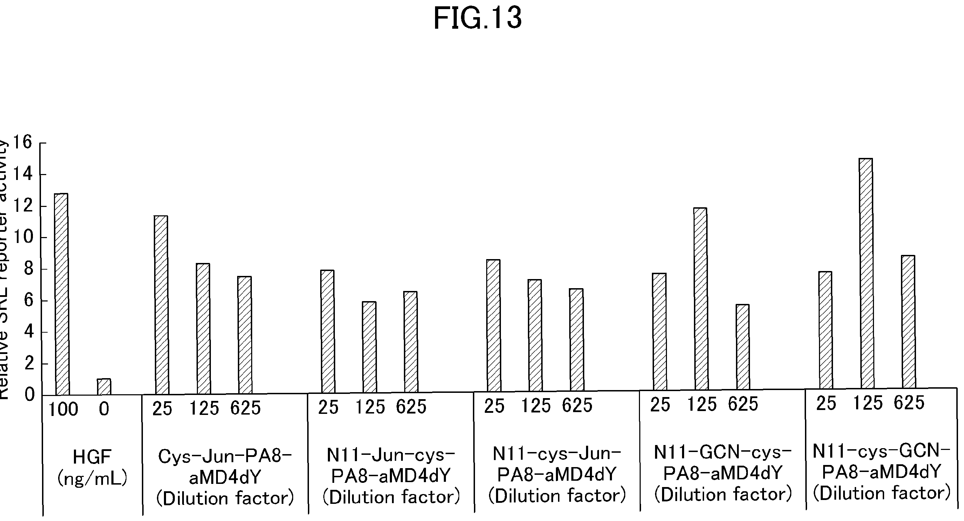

Fig. 13 is a graph showing activity evaluation of dimerized cyclic peptides (cys-Jun-PA8-aMD4dY, N11-Jun-cys-PA8-aMD4dY, N11-cys-Jun-PA8-aMD4dY, N11-GCN4-cys-PA8-aMD4dY, and N11-cys-GCN4-PA8-aMD4dY) synthesized with HGF. The vertical axis represents relative SRE receptor activity, and the horizontal axis represents the concentration for HGF, and for the synthesized dimerized cyclic peptides the dilution factor of the reaction solution of the cell-free expression system with respect to the medium subjected to activity evaluation.

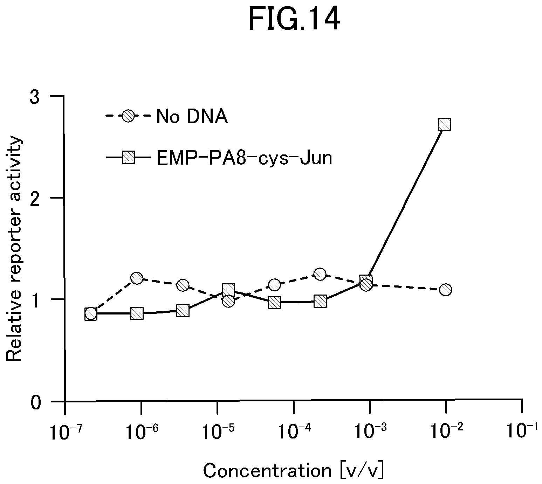

Fig. 14 is a graph showing activity evaluation of a synthesized dimerized cyclic peptide (EMP-cys-PA8-Jun). The vertical axis represents the relative receptor activity, and the horizontal axis represents the concentration of the reaction solution of the cell-free expression system with respect to the medium subjected to activity evaluation. The negative control used was a cell-free expression system without the addition of the EMP-cys-PA8-Jun synthesis gene (No DNA).

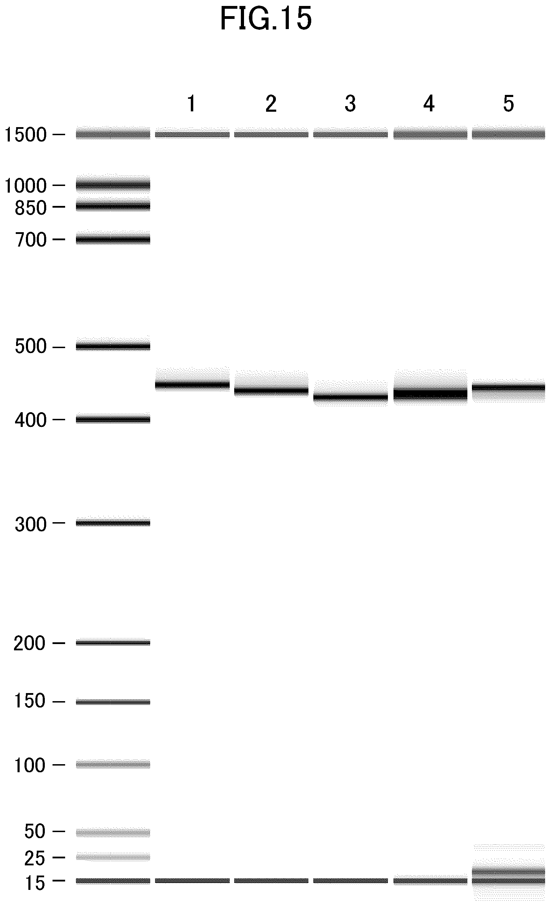

Fig. 15 shows the results of DNA length analysis between aMD4dY-Jun-cys-N11-SecM-GS3, TPO-Jun-cys-N11-SecM-GS3, and EMP'-Jun-cys-N11-SecM-GS3 used for DNA library construction and the DNA library before and after ribosome display. Lane 1 is a template DNA for aMD4dY-Jun-cys-N11-SecM-GS3, Lane 2 is a template DNA for TPO-Jun-cys-N11-SecM-GS3, Lane 3 is a template DNA for EMP'-Jun-cys-N11-SecM-GS3, Lane 4 is a DNA mix, and Lane 5 is a DNA mix2.

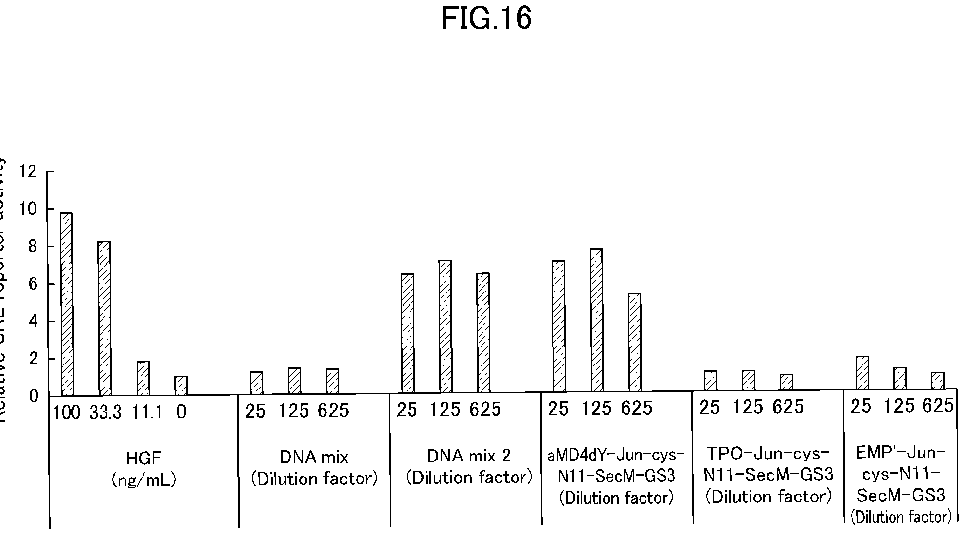

Fig. 16 is a graph showing activity evaluation of cell-free translation products of HGF and DNA mix, cell-free translation products of DNA mix2, cell-free translated aMD4dY-Jun-cys-N11-SecM-GS3, cell-free translated TPO-Jun-cys-N11-SecM-GS3, and cell-free translated EMP'-Jun-cys-N11-SecM-GS3. The vertical axis represents relative receptor activity, and the horizontal axis represents the concentration for HGF, and for the cell-free translated cyclic peptides the dilution factor of the reaction solution of the cell-free expression system with respect to the medium subjected to activity evaluation.

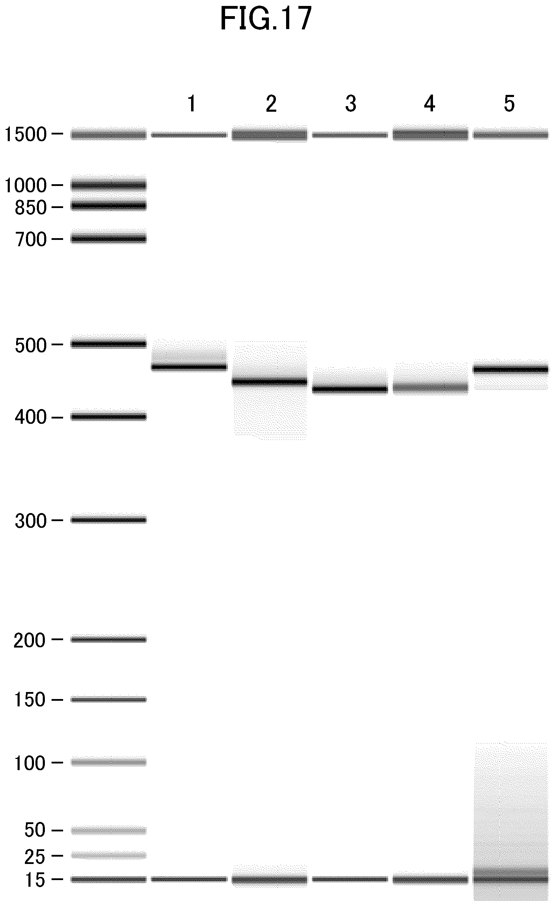

Fig. 17 shows the results of DNA length analysis between EMP-Jun-cys-N11-SecM-GS3, aMD4dY-Jun-cys-N11-SecM-GS3, and TPO-Jun-cys-N11-SecM-GS3 used for DNA library construction and the DNA library before and after ribosome display. Lane 1 is a template DNA for EMP-Jun-cys-N11-SecM-GS3, Lane 2 is a template DNA for aMD4dY-Jun-cys-N11-SecM-GS3, Lane 3 is a template DNA for TPO-Jun-cys-N11-SecM-GS3, Lane 4 is a DNA mix, and Lane 5 is a DNA mix2.

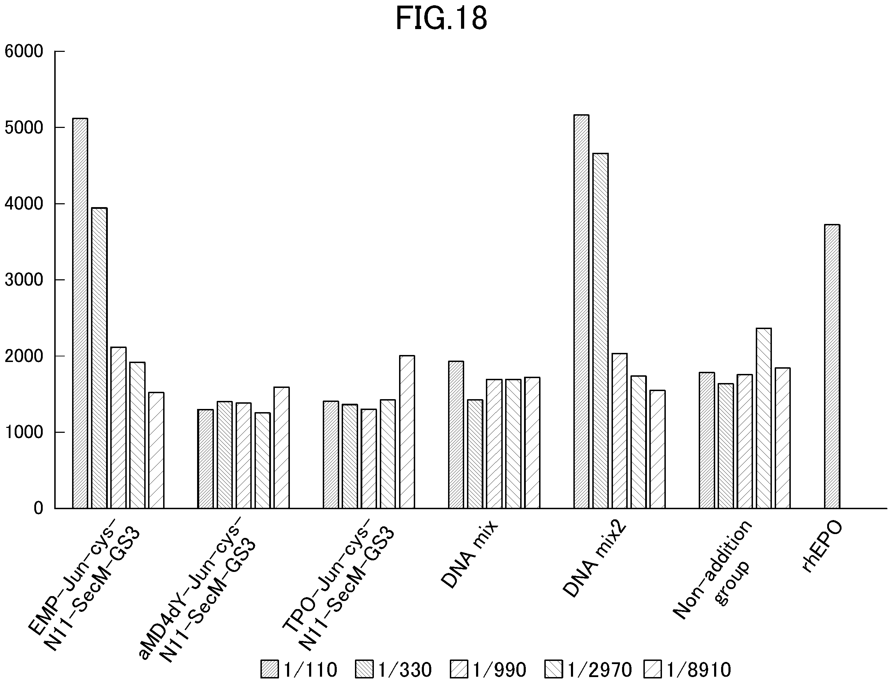

Fig. 18 is a graph showing activity evaluation of cell-free translated EMP-Jun-cys-N11-SecM-GS3, cell-free translated aMD4dY-Jun-cys-N11-SecM-GS3, cell-free translated TPO-Jun-cys-N11-SecM-GS3, cell-free translation products of DNA mix, cell-free translation products of DNA mix2, DNA-free non-addition group, and recombinant human EPO (rhEPO). For the cell-free translated cyclic peptides and DNA-free non-addition group, concentrations of the reaction solution of the cell-free expression in the medium subjected to activity evaluation are shown as the following 5 consecutive dilutions (1/110, 1/330, 1/990, 1/2970, and 1/8910). For rhEPO, 50 µg/mL rhEPO was added to the medium subjected to activity evaluation at a concentration of 1/110. The vertical axis represents chemiluminescence intensity (LU).

### Description of Embodiments

An embodiment of the present invention relates to molecules including cyclic peptides. A molecule including such a cyclic peptide is in particular a polypeptide including a cyclic peptide, a dimerization domain containing a cysteine residue and a leucine zipper region, and a peptide linker present between the cyclic peptide and the dimerization domain. This polypeptide can be used in the method for screening dimerized cyclic peptides and libraries therefor described herein. In the polypeptide, each amino acid may be an L-amino acid or a D-amino acid, and may be natural or non-natural, and each amino acid is preferably an α-, β-, or γ-amino acid, more preferably an α-amino acid.

In an embodiment, such a polypeptide may have, for example, a structure containing "cyclic peptide" - "peptide linker" - "cysteine residue" - "leucine zipper region" from the N-terminal side, a structure containing "cyclic peptide" - "peptide linker" - "leucine zipper region" - "cysteine residue" from the N-terminal side, a structure containing "cysteine residue" - "leucine zipper region" - "peptide linker" - "cyclic peptide" from the N-terminal side, or a structure containing "leucine zipper region" - "cysteine residue" - "peptide linker" - "cyclic peptide" from the N-terminal side. Furthermore, the polypeptide may contain any amino acid sequence composed of at least one amino acid residue on the N-terminal side or the C-terminal side, or between "cyclic peptide" and "peptide linker", between "peptide linker" and "cysteine residue", between "peptide linker" and "leucine zipper region", or between "cysteine residue" and "leucine zipper region". Any such amino acid sequence may include, but is not particularly limited to, a tag sequence, an enzyme recognition sequence, or a sequence useful for enhancing the expression of a polypeptide. Examples of tag sequences include N11 tag, FLAG tag, PA tag, and histidine tag, and the tag sequence is preferably present on the N-terminal side of the above polypeptide. Examples of enzyme recognition sequences include protease cleavage sequences and sugar chain modification sequences. The amino acid sequence that can be contained between "cyclic peptide" and "peptide linker", between "peptide linker" and "cysteine residue", between "peptide linker" and "leucine zipper region", or between "cysteine residue" and "leucine zipper region" includes, but is not particularly limited to, one composed of one or more glycines, preferably one composed of 1 to 5 glycines.

In a polypeptide including a cyclic peptide, a dimerization domain containing a cysteine residue and a leucine zipper region, and a peptide linker present between the cyclic peptide and the dimerization domain, the cyclic peptide is not particularly limited, but for example may have a molecular weight of 250 or more, 500 or more, 800 or more, or 1000 or more, and may have a molecular weight of 10000 or less, 9000 or less, 8000 or less, or 7000 or less. In addition, the cyclic peptide is not particularly limited, but for example may be composed of 3 or more, 6 or more, 10 or more, or 12 or more amino acid residues, and may be composed of 50 or less, 45 or less, 40 or less, or 35 or less amino acid residues.

The cyclic peptide may form a cyclic structure, for example, through a disulfide bond between two cysteine residues, although there is no particular limitation on the binding mode in which it forms a cyclic structure.

Examples of the cyclic peptide include, but are not limited to, FGFR-binding peptides, c-Met (HGFR)-binding peptides, erythropoietin receptor-binding peptides, thrombopoietin receptor-binding peptides, albumin-binding peptides, EGFR-binding peptides, VEGFR-binding peptides, PDGFR-binding peptides, Axl-binding peptides, PDGFR-binding peptides, SCFR-binding peptides, Flt-3-binding peptides, c-Ret-binding peptides, ROR-binding peptides, Tie-binding peptides, NGFR-binding peptides, Insulin receptor-binding peptides, EphR-binding peptides, Alk-binding peptides, DDR-binding peptides, TGFBR-binding peptides, Activin receptor-binding peptides, BMP receptor-binding peptides, interleukin receptor-binding peptides, T-cell receptor-binding peptides, transferrin receptor-binding peptides, lipoprotein receptor-binding peptides, ubiquitin ligase-binding peptides, antibody-binding peptides, complement-binding peptides, GPCR-binding peptides, ion channel-binding peptides, virus-binding peptides, or peptides expected to act as these peptides.

Specific examples of cyclic peptides that bind to c-Met (also referred to simply as Met) include, but are not limited to, the following cyclic peptides.

A cyclic peptide comprising or composed of an amino acid sequence selected from the following (a) to (i) and forming a cyclic structure with a disulfide bond.
(a) CYRQFNRRTHEVWNLDC (SEQ ID NO: 1)
(b) CRQFNRRTHEVWNLDC (SEQ ID NO: 2) (Also referred to herein as aMD4dY)
(c) CYWYYAWDQTYKAFPC (SEQ ID NO: 3)
(d) CWYYAWDQTYKAFPC (SEQ ID NO: 4)
(e) CYISWNEFNSPNWRFITC (SEQ ID NO: 5)
(f) CISWNEFNSPNWRFITC (SEQ ID NO: 6)
(g) A peptide in which one or two amino acid residues other than a cysteine residue are substituted, deleted, or added in any amino acid sequence of (a) to (f) and which binds to c-Met protein.
(h) A peptide which is composed of an amino acid sequence having 90% or more sequence identity with any amino acid sequence of (a) to (f) but bearing cysteine residues at both ends and which binds to c-Met protein. For example, the sequence identity may be 92% or greater, 95% or greater, or 98% or greater. Herein, amino acid sequence homology can be calculated using known analysis tools, and can be calculated, for example, using the homology algorithm BLAST (Basic local alignment search tool) of the National Center for Biotechnology Information (NCBI). In addition, for calculation of amino acid sequence homology, default (initial setting) parameters in the analysis tool may be used. (i) A peptide in which at least one amino acid other than cysteine residues at both ends of any amino acid sequence of (a) to (h) is modified. Here, amino acid modification is phosphorylation, methylation, acetylation, adenylation, ADP-ribosylation, or sugar chain addition.

Met is an HGF receptor and a tyrosine kinase receptor, and such a cyclic peptide that binds to Met (HGFR) is known to function as a Met (HGFR) agonist by dimerization.

In the expression "substitution, deletion, or addition" as described herein, substitution means substitution with a known amino acid, preferably conservative amino acid substitution. The "conservative amino acid substitution" is a substitution in which an amino acid residue is replaced by another amino acid residue having a side chain R group with similar chemical properties (for example, charge or hydrophobicity). Examples of amino acid groups having side chains with similar chemical properties include: glycine, alanine, valine, leucine, and isoleucine with aliphatic side chains; serine and threonine having aliphatic hydroxyl side chains; asparagine and glutamine having amide-containing side chains; phenylalanine, tyrosine, and tryptophan having aromatic side chains; lysine, arginine, and histidine having basic side chains; and aspartic acid and glutamic acid having acidic side chains. Each amino acid introduced by substitution may be an L-amino acid or a D-amino acid, and may be a natural amino acid or a non-natural amino acid, but preferably an amino acid that constitutes protein in humans and animals.

In the expression "substitution, deletion, or addition" as described herein, addition means adding a known amino acid to the end of an amino acid sequence or inserting a known amino acid between amino acid residues of an amino acid sequence. Each amino acid introduced by addition may be an L-amino acid or a D-amino acid, and may be a natural amino acid or a non-natural amino acid, but preferably an amino acid that constitutes protein in humans and animals.

In addition, specific examples of cyclic peptides that bind to an erythropoietin receptor include, but are not limited to, the following cyclic peptides.

A cyclic peptide comprising or composed of an amino acid sequence selected from the following (j) to (n) and forming a cyclic structure with a disulfide bond.
(j) GGLYACHMGPMTWVCQPLRG (SEQ ID NO: 7)
(k) CISWNEFNSPNWRFITC (SEQ ID NO: 8)
(l) A peptide in which one or two amino acid residues other than a cysteine residue are substituted, deleted, or added in any amino acid sequence of (j) or (k) and which binds to an erythropoietin receptor.
(m) A peptide which is composed of an amino acid sequence having 90% or more sequence identity with any amino acid sequence of (j) or (k) but bearing cysteine residues at the positions as they are in (j) or (k) and which binds to an erythropoietin receptor. For example, the sequence identity may be 92% or greater, 95% or greater, or 98% or greater.
(n) A peptide in which at least one amino acid other than cysteine residues of any amino acid sequence of (j) to (m) is modified. Here, amino acid modification is phosphorylation, methylation, acetylation, adenylation, ADP-ribosylation, or sugar chain addition.

In a polypeptide including a cyclic peptide, a dimerization domain containing a cysteine residue and a leucine zipper region, and a peptide linker present between the cyclic peptide and the dimerization domain, the peptide linker is not particularly limited, but may have a molecular weight of 10,000 or less and may be composed of 50 or less amino acid residues.

In addition, in an embodiment, the peptide linker may have a molecular weight of 10,000 or less and may be composed of 50 or less amino acid residues, and at least 90%, at least 95%, or 100% of the amino acid sequence may be composed of amino acid residues selected from alanine (A), proline (P), and serine (S). In this case, proline residues may constitute more than 4% and less than 40% of the amino acid sequence of the peptide linker. For example, proline residues may constitute more than about 4%, more than about 5%, more than about 6%, more than about 8%, more than about 10%, more than about 15%, or more than about 20%, and may constitute less than about 40%, or less than about 35%, in the amino acid sequence of the peptide linker. In addition, alanine and serine residues may each independently constitute more than about 4%, more than about 10%, or more than about 20%, and may constitute less than about 50%.

The term "about X%" as described herein is not limited to the number X, but also includes values of 10% to 20% more or 10% to 20% less. For example, the term "about 10%" also relates to 11% or 12% and 9% or 8%, respectively.

In the amino acid sequence of the peptide linker, amino acid residues different from alanine, serine, and proline can be selected from the group consisting of Arg, Asn, Asp, Cys, Gln, Glu, Gly, His, Ile, Leu, Lys, Met, Phe, Thr, Trp, Tyr, and Val. Such amino acid residues different from alanine, serine, and proline preferably do not have hydrophobic side chains such as Val, Ile, Leu, Met, Phe, Tyr, or Trp, and/or do not have charged side chains such as Lys, Arg, Asp, or Glu.

In an embodiment, the peptide linker may have a molecular weight of 10,000 or less and may be composed of 50 or less amino acid residues, and at least 90%, at least 95%, or 100% of the amino acid sequence may be composed of amino acid residues selected from alanine (A) and proline (P). In this case, proline residues may constitute more than 10% and less than 75% of the amino acid sequence of the peptide linker. For example, proline residues may constitute more than about 10%, more than about 12%, more than about 14%, more than about 18%, more than about 20%, more than about 22%, more than about 23%, more than about 24%, or more than about 25%, and may constitute less than about 75%, less than about 70%, less than about 65%, less than about 60%, less than about 55%, less than about 50%, less than about 48%, less than about 46%, less than about 44%, less than about 42%, less than about 41%, less than about 40%, less than about 39%, less than about 38%, less than about 37%, less than about 36%, or less than about 35%, in the amino acid sequence of the peptide linker. In addition, alanine residues may constitute more than about 25%, more than about 30%, more than about 35%, more than about 40%, more than about 45%, more than about 50%, more than about 52%, more than about 54%, more than about 56%, more than about 58%, more than about 59%, more than about 60%, more than about 61%, more than about 62%, more than about 63%, more than about 64%, or more than about 65%, and may constitute less than about 90%, less than about 88%, less than about 86%, less than about 84%, less than about 82%, less than about 80%, less than about 79%, less than about 78%, less than about 77%, less than about 76%, or less than about 75%.

In the amino acid sequence of the peptide linker, amino acid residues different from alanine, serine, and proline can be selected from the group consisting of Arg, Asn, Asp, Cys, Gln, Glu, Gly, His, Ile, Leu, Lys, Met, Phe, Thr, Trp, Tyr, and Val. Such amino acid residues different from alanine, serine, and proline preferably do not have hydrophobic side chains such as Val, Ile, Leu, Met, Phe, Tyr, or Trp, and/or do not have charged side chains such as Lys, Arg, Asp, or Glu.

In an embodiment, the peptide linker contains multiple amino acid repeats, and the amino acid repeats are composed of alanine (A), proline (P), and serine (S), and the number of consecutive identical amino acid residues in the amino acid repeats may be 6 residues or less, 5 residues or less, 4 residues or less, or 3 residues or less.

In an embodiment, the peptide linker contains multiple amino acid repeats, and the amino acid repeats are composed of alanine (A) and proline (P), and the number of consecutive identical amino acid residues in the amino acid repeats may be 6 residues or less, 5 residues or less, 4 residues or less, or 3 residues or less.

In an embodiment, the peptide linker may contain two or more repeat sequences represented by AP or AAP.

In an embodiment, the peptide linker may contain a sequence composed of glycine (G) and serine (S). In this case, the peptide linker may repeatedly contain an amino acid sequence selected from the group consisting of GGGGS, GGGS, GGS, and GS.

In an embodiment, the peptide linker may contain consecutive glycines or may be composed solely of glycines.

In an embodiment, the peptide linker may repeatedly contain the amino acid sequence represented by EAAAK.

In one embodiment, the amino acid sequence of the peptide linker is preferably one composed of A, P, and S, one composed of A and P, or repeats of the sequence represented by GGGGS, but is not limited to these.

In an embodiment, the length of the peptide linker may be composed of 50 or less amino acid residues. For example, the peptide linker may be composed of 49 or less, 40 or less, 35 or less, 30 or less, or 25 or less amino acid residues.

In an embodiment, the length of the peptide linker may be composed of 1 or more amino acid residues. For example, the peptide linker may be composed of 4 or more, 8 or more, 12 or more, or 16 or more amino acid residues.

In an embodiment, specific examples of the peptide linker include, but are not limited to, those containing the following amino acid sequences and those composed of the following amino acid sequences. Amino acid sequences selected from AAPAAPAPAAPAAPAPAAPA (SEQ ID NO: 9), AAPAAPAPAAPAAPAPAAPAAP (SEQ ID NO: 10), AAPAAPAPAAPAAP (SEQ ID NO: 11), AAPAAAPAPAAPAAPAPAAP (SEQ ID NO 12), AAAPAAAPAAAPAAAPAAAP (SEQ ID NO 13), AAPAAPAAPAAPAAPAAPAAPAAP (SEQ ID NO 14), APAAAPAPAAAPAPAAAPAPAAAP (SEQ ID NO 15), AAAPAAPAAPPAAAAPAAPAAPPA (SEQ ID NO 16), and APAPAPAPAPAPAPAPAPAP (SEQ ID NO 17), ASPAAPAPASPAAPAPSAPA (SEQ ID NO 18), AAPASPAPAAPSAPAPAAPS (SEQ ID NO 19), APSSPSPSAPSSPSPASPSS (SEQ ID NO 20), SAPSSPSPSAPSSPSPASPS (SEQ ID NO 21), SSPSAPSPSSPASPSPSSPA (SEQ ID NO 22), AASPAAPSAPPAAASPAAPSAPPA (SEQ ID NO 23), ASAAAPAAASAAASAPSAAA (SEQ ID NO 24), AAPAAPAPAAPAAPAP (SEQ ID NO: 25), AAPAAPAPAAPAAPAPAAPAAPAPAAPAAPAPAAPAAPAPAAPAAPAPA (SEQ ID NO: 26), and SAPSPSSAPASASAPASPASASASASPASSPASASSASPAASSASPASS (SEQ ID NO: 27), and circularly permuted sequences thereof.

Taking AAPAAPAPAAPAAPAPAAPA (SEQ ID NO: 9) as an example, circularly permuted sequences can be readily prepared, for example, by removing the first alanine of the above sequence and adding another alanine to the end of the above sequence. Such a circularly permuted sequence of SEQ ID NO: 9 becomes APAAPAPAAPAAPAPAAPAA (SEQ ID NO: 28). In addition, non-limiting examples of circularly permuted sequences of SEQ ID NO: 7 whose order of amino acid residues has been circularly changed include the following. PAAPAPAAPAAPAPAAPAAA (SEQ ID NO: 29), AAPAPAAPAAPAPAAPAAAP (SEQ ID NO: 30), APAPAAPAAPAPAAPAAAPA (SEQ ID NO: 31), PAPAAPAAPAPAAPAAAPAA (SEQ ID NO: 32), APAAPAAPAPAAPAAAPAAP (SEQ ID NO: 33), PAAPAAPAPAAPAAAPAAPA (SEQ ID NO: 34), AAPAAPAPAAPAAAPAAPAP (SEQ ID NO: 35), APAAPAPAAPAAAPAAPAPA (SEQ ID NO: 36), and PAAPAPAAPAAAPAAPAPAA (SEQ ID NO: 37).

In an embodiment, specific examples of the peptide linker include, but are not limited to, those containing the following amino acid sequences and those composed of the following amino acid sequences. Amino acid sequences selected from AAPAAPAPAA (SEQ ID NO: 38), AAPAAPAP (SEQ ID NO: 39), and AAPAAPAPA (SEQ ID NO: 40), and circularly permuted sequences thereof.

In a polypeptide including a cyclic peptide, a dimerization domain containing a cysteine residue and a leucine zipper region, and a peptide linker present between the cyclic peptide and the dimerization domain, the dimerization domain may have only one, or two or more cysteine residues. Preferably, the dimerization domain has only one cysteine residue.

The leucine zipper region in the dimerization domain is a region that forms an α-helix and has a leucine residue every seven residues. The leucine zipper region is not particularly limited, but may be composed of, for example, 60 or less, 45 or less, 35 or less, or 25 or less amino acid residues.

The leucine zipper region in the dimerization domain is not particularly limited as long as it forms a dimer. Examples thereof include leucine zipper domains of c-Jun protein, GCN4 protein, or CEBPα protein, and JunΔhep with deleted heparin-binding ability by substituting positively charged amino acids of c-Jun leucine zipper domain with glutamine (M. D. Ballinger et al., Nat. Biotechnol. 17, 1199-1204 (1999), the contents of which are incorporated herein by reference). Specific examples of the leucine zipper region include, but are not limited to, those containing the following amino acid sequences and those composed of the following amino acid sequences. RIARLEEKVKTLKAQNSELASTANMLREQVAQLKQKVMN (SEQ ID NO: 41), QIAQLEEKVQTLQAQNSELASTANMLREQVAQLKQKVMN (SEQ ID NO: 42), RMKQLEDKVEELLSKNYBLENEVARLKKLVGER (SEQ ID NO: 43), and RNVETQQKVLELTSDNDRLRKRVEQLSRELDTLRGIFRQ (SEQ ID NO: 44).

In an embodiment, the leucine zipper region in the dimerization domain may have its heparin binding ability removed.

The polypeptide as described herein, which includes a cyclic peptide, a dimerization domain containing a cysteine residue and a leucine zipper region, and a peptide linker present between the cyclic peptide and the dimerization domain, is not particularly limited, but may have, for example, a molecular weight of 3000 or more, 4000 or more, 5000 or more, or 6000 or more, and a molecular weight of 20000 or less, 18000 or less, 16000 or less, or 14000 or less. In addition, the polypeptide is not particularly limited, but may be composed of, for example, 30 or more, 40 or more, 50 or more, or 60 or more amino acid residues, and may be composed of 200 or less, 180 or less, 160 or less, or 140 or less amino acid residues.

The polypeptide as described herein, which includes a cyclic peptide, a dimerization domain containing a cysteine residue and a leucine zipper region, and a peptide linker present between the cyclic peptide and the dimerization domain, can be prepared using known techniques based on a nucleic acid encoding the polypeptide. In addition, the polypeptide can dimerize through non-covalent interactions at the leucine zipper region in the dimerization domain and disulfide bonds between cysteine residues in the dimerization domain. Disulfide bonds between cysteine residues contribute to tight dimerization and can greatly affect the activity of dimerized cyclic peptides.

A nucleic acid encoding the polypeptide can be DNA or RNA (mRNA). Synthesis of such nucleic acids can be appropriately carried out by those skilled in the art using conventional synthesis techniques based on an amino acid sequence of a desired polypeptide.

In an embodiment, a nucleic acid encoding the polypeptide may have a promoter sequence necessary for cell-free expression (such as T7 promoter sequence) and a ribosome binding sequence on the 5'-side. In addition, the nucleic acid may have a stop codon and an untranslated region on the 3'-side.

Conventionally, as a method for obtaining a peptide dimer formed by dimerizing a cyclic peptide in one pot using a biosynthetic system, a method for expressing from a gene a molecular format in which two cyclic peptides are linked with a linker was used. However, preparation of a template gene for such synthesis requires changing sequences at two locations to one encoding a cyclic peptide, and requires total gene synthesis. On the other hand, the polypeptide as described herein dimerizes after synthesis of the polypeptide, and the nucleic acid used to synthesize such a polypeptide has a sequence at only one location encoding a cyclic peptide. Therefore, in the invention as described herein, nucleic acid synthesis and polypeptide synthesis can be performed in a rapid and uncomplicated manner.

An embodiment of the present invention relates to a method for preparing dimerized cyclic peptides. Methods for preparing such dimerized cyclic peptides include, for example, synthesizing a polypeptide containing a cyclic peptide, a dimerization domain containing a cysteine residue and a leucine zipper region, and a peptide linker present between the cyclic peptide and the dimerization domain in a cell-free expression system to prepare a dimer of the polypeptide, and the dimer may be a homodimer or a heterodimer. Here, cyclic peptides, dimerization domains (cysteine residues and leucine zipper regions), and peptide linkers are as described above herein.

Protein synthesis in a cell-free expression system is a technique known to those skilled in the art, and may involve the use of a cell extract or a commercially available cell-free expression reagent. Components included in the cell-free expression system include a template nucleic acid (DNA or mRNA) encoding a desired polypeptide, proteins necessary for transcription, translation, energy regeneration, and post-translational modification (such as RNA polymerase, ribosome, aminoacyl-tRNA synthetase, translation initiation factor, translation elongation factor, translation termination factor, ribosome regeneration factor, nucleoside diphosphate kinase, adenosine kinase, creatine kinase, prolyl isomerase, disulfide bond isomerase, and chaperone), tRNAs, aminoacyl-tRNAs, natural amino acids, non-natural amino acids, NTPs, buffers, and the like.

Examples of commercially available cell-free expression reagents include PUREfrex 2.0 (Gene Frontier) and PURExpress In Vitro Protein Synthesis Kit (New England BioLabs).

An embodiment of the present invention relates to a method for screening dimerized cyclic peptides. Methods for screening such dimerized cyclic peptides include, for example, the following step (1A) or (1B) and step (2).
(1A) Synthesizing at least one polypeptide including
   a cyclic peptide,
   a dimerization domain containing a cysteine residue and a leucine zipper region, and
   a peptide linker present between the cyclic peptide and the dimerization domain
   in a cell-free expression system to prepare a dimer of the polypeptide, or
(1B) providing a dimer of at least one polypeptide including
   a cyclic peptide,
   a dimerization domain containing a cysteine residue and a leucine zipper region, and
   a peptide linker present between the cyclic peptide and the dimerization domain
   obtained by synthesizing the polypeptide in a cell-free expression system, and
(2) measuring activity based on binding of the dimer to a target molecule.

The method for screening dimerized cyclic peptides may further include screening or selecting the dimer (dimerized cyclic peptide) based on the activity measured in step (2).

In addition, an embodiment of the present invention relates to a method for obtaining dimerized cyclic peptides having activity based on binding to a target molecule by measuring the activity based on the binding to the target molecule by the method for screening dimerized cyclic peptides.

In the method for screening dimerized cyclic peptides, step (1A) is as described herein above for the method for preparing dimerized cyclic peptides, and step (1B) provides a dimerized cyclic peptide (polypeptide dimer) obtained by a method as described herein above. As to the "at least one polypeptide including a cyclic peptide, a dimerization domain containing a cysteine residue and a leucine zipper region, and a peptide linker present between the cyclic peptide and the dimerization domain" in steps (1A) and (1B), the content of the "polypeptide including a cyclic peptide, a dimerization domain containing a cysteine residue and a leucine zipper region, and a peptide linker present between the cyclic peptide and the dimerization domain" described herein above can be applied as it is. The at least one polypeptide in steps (1A) and (1B) may include a plurality of different polypeptides expected to have binding affinity with a target molecule.

In addition, "polypeptide dimer" as described herein is synonymous with "dimerized cyclic peptide" as described herein above.

In step (2), "target molecule" means a molecule to which the cyclic peptide or dimerized cyclic peptide described herein binds or is expected to bind. The target molecule is preferably a receptor, more preferably a receptor that is activated by dimerization, such as proliferation factor receptors, growth factor receptors, and cytokine receptors. Specific examples of target molecules include, but are not limited to, FGFR, c-Met (HGFR), erythropoietin receptors, thrombopoietin receptors, albumin, EGFR, VEGFR, PDGFR, Axl, PDGFR, SCFR, Flt-3, c-Ret, ROR, Tie, NGFR, Insulin receptors, EphR, Alk, DDR, TGFBR, Activin receptors, BMP receptors, interleukin receptors, T-cell receptors, transferrin receptors, lipoprotein receptors, ubiquitin ligases, antibodies, complements, GPCRs, ion channels, and viruses.

In step (2), the "activity based on binding of the dimer to a target molecule" refers to an action resulting from binding of the dimer to the target molecule and/or strength of the action, and can include both agonistic activity and antagonistic activity. For example, activity can include receptor activity, signaling activity, apoptotic activity, and the like.

The measurement in step (2) can be carried out using known techniques, including bioluminescence assays such as luciferase reporter assay, receptor dimerization assays such as PathHunter^{®} Receptor Dimerization assay, or fluorescence resonance energy transfer (FRET) measurements, or cross-linking with chemical linkers and dimerization analysis by SDS-PAGE, or the like.

For the measurement in step (2), the cell-free expression reaction solution obtained in step (1) may be used as it is, or may be used after being diluted as appropriate.

In an embodiment, in the method for screening dimerized cyclic peptides, in step (1), at least one polypeptide includes a plurality of different polypeptides expected to have binding affinity with a target molecule, and step (2) further includes measuring activity of dimers of the plurality of different polypeptides based on binding to the target molecule.

In an embodiment, in the method for screening dimerized cyclic peptides, step (1) further includes creating a library including a plurality of at least one polypeptide or dimer thereof, and the plurality of polypeptides include different cyclic peptides. Here, a library including a plurality of at least one polypeptide or dimer thereof may include, for example, 2 or more, 8 or more, 16 or more, 32 or more, 48 or more, 96 or more, 200 or more, 1000 or more, 10000 or more, 10⁵ or more, or 10⁶ or more kinds of the polypeptide or dimers thereof. In addition, a library including a plurality of at least one polypeptide or dimer thereof may include, for example, 10¹⁵ or less, 10¹⁴ or less, or 10¹³ or less kinds of the polypeptide or dimers thereof.

The term "library" as described herein refers to a collection including a plurality of different substances (such as different polypeptides or different nucleic acids).

An embodiment of the invention relates to a method for creating a library. A method for creating such a library can be, for example, a method for creating a library including a plurality of polypeptides or dimers of the plurality of polypeptides, including synthesizing the plurality of polypeptides in a cell-free expression system, wherein the plurality of polypeptides each include a cyclic peptide, a dimerization domain containing a cysteine residue and a leucine zipper region, and a peptide linker present between the cyclic peptide and the dimerization domain, and the plurality of polypeptides include different cyclic peptides.

In this method for creating a library, the step of "synthesizing a plurality of polypeptides in a cell-free expression system" is as described herein above for the method for preparing dimerized cyclic peptides. Also, the content "polypeptide including a cyclic peptide, a dimerization domain containing a cysteine residue and a leucine zipper region, and a peptide linker present between the cyclic peptide and the dimerization domain" as described herein above can be applied as it is to the content for the polypeptides, "the plurality of polypeptides each include a cyclic peptide, a dimerization domain containing a cysteine residue and a leucine zipper region, and a peptide linker present between the cyclic peptide and the dimerization domain".

An embodiment of the present invention relates to a library for screening dimerized cyclic peptides. Such a library includes, for example, a plurality of polypeptides or dimers of the plurality of polypeptides, or a plurality of nucleic acids encoding the plurality of polypeptides, and the plurality of polypeptides each include a cyclic peptide, a dimerization domain containing a cysteine residue and a leucine zipper region, and a peptide linker present between the cyclic peptide and the dimerization domain, and the plurality of polypeptides include different cyclic peptides.

In this library, the content "polypeptide including a cyclic peptide, a dimerization domain containing a cysteine residue and a leucine zipper region, and a peptide linker present between the cyclic peptide and the dimerization domain" as described herein above can be applied as it is to the content for the polypeptides, "the plurality of polypeptides each include a cyclic peptide, a dimerization domain containing a cysteine residue and a leucine zipper region, and a peptide linker present between the cyclic peptide and the dimerization domain".

Such a library may include, for example, 2 or more, 8 or more, 16 or more, 32 or more, 48 or more, 96 or more, 200 or more, 1000 or more, 10000 or more, 10⁵ or more, or 10⁶ or more kinds of polypeptides or dimers of the polypeptides. In addition, such a library may include, for example, 10¹⁵ or less, 10¹⁴ or less, or 10¹³ or less kinds of polypeptides or dimers of the polypeptides.

Alternatively, such a library may include, for example, 2 or more, 8 or more, 16 or more, 32 or more, 48 or more, 96 or more, 200 or more, 1000 or more, 10000 or more, 10⁵ or more, or 10⁶ or more kinds of nucleic acids encoding a plurality of polypeptides. In addition, such a library may include, for example, 10¹⁵ or less, 10¹⁴ or less, or 10¹³ or less kinds of nucleic acids encoding a plurality of polypeptides. The "nucleic acids encoding polypeptides" are as described herein above.

An embodiment of the present invention relates to a method for screening dimerized cyclic peptides. Such methods for screening dimerized cyclic peptides include, for example, the following steps (1') to (5') as methods different from those described above.
(1') Obtaining one or a plurality of polypeptides having binding affinity with a target molecule from a library including a plurality of displayed polypeptides, wherein the plurality of displayed polypeptides each include
   a cyclic peptide,
   a dimerization domain containing a cysteine residue and a leucine zipper region, and
   a peptide linker present between the cyclic peptide and the dimerization domain, and
   the plurality of displayed polypeptides include different cyclic peptides,
(2') recovering a nucleic acid associated with the polypeptides obtained in step (1'),
(3') amplifying the nucleic acid recovered in step (2'),
(4') synthesizing a polypeptide including
   a cyclic peptide,
   a dimerization domain containing a cysteine residue and a leucine zipper region, and
   a peptide linker present between the cyclic peptide and the dimerization domain
   in a cell-free expression system using the nucleic acid amplified in step (3') to prepare a dimer of the polypeptide, and
(5') measuring activity based on binding of the dimer to a target molecule.

A method for screening dimerized cyclic peptides including such steps (1') to (5') makes it possible to provide screening for dimerized cyclic peptides having activity based on binding to a target molecule, which can be seamlessly assessed from acquiring displayed candidate polypeptides.

A method for screening dimerized cyclic peptides including such steps (1') to (5') may further include screening or selecting the dimer (dimerized cyclic peptide) based on the activity measured in step (5').

In addition, an embodiment of the present invention relates to a method for obtaining dimerized cyclic peptides having activity based on binding to a target molecule by measuring the activity based on the binding to the target molecule by the method for screening dimerized cyclic peptides, including such steps (1') to (5').

Step (1') is a step of obtaining one or a plurality of polypeptides having binding affinity with the target molecule from a library including a plurality of displayed polypeptides. Here, a "library including a plurality of displayed polypeptides" can be created using known display techniques. Examples of such display techniques include ribosome display, mRNA display, phage display, bacterial display, and yeast display. Specifically, introducing the SecM sequence into a template DNA sequence facilitates the production of ribosome-polypeptide (fusion protein)-mRNA complexes, making it possible to create a library including a plurality of displayed polypeptides in an uncomplicated manner. However, the sequences and display methods used are not limited to this.

In step (1'), the library may include 2 or more, 8 or more, 16 or more, 32 or more, 48 or more, 96 or more, 200 or more, 1000 or more, 10000 or more, 10⁵ or more, 10⁶ or more, 10⁷ or more, 10⁸ or more, 10⁹ or more, or 10¹⁰ or more kinds of displayed polypeptides. Also, such a library can include 10¹⁵ or less, 10¹⁴ or less, or 10¹³ or less kinds of polypeptides or dimers of the polypeptides.

In addition, in step (1'), "target molecule" as well as "cyclic peptides", "dimerization domain containing a cysteine residue and a leucine zipper region", and "peptide linker present between the cyclic peptide and the dimerization domain" of "plurality of displayed polypeptides" are as described herein above.

In step (1'), obtaining one or a plurality of polypeptides having binding affinity with a target molecule can be carried out using known techniques, including affinity selection on recombinant target molecules or on cells expressing the target molecules.

Step (2') is a step of recovering a nucleic acid associated with the polypeptides obtained in step (1'). This can be carried out using known techniques, such as a recovery method using magnetic beads, a recovery method using an ELISA plate, a recovery method using cell pellets, a recovery method using nanodroplets, and a technique using a microchannel.

Step (3') is a step of amplifying the nucleic acid recovered in step (2'). This can be carried out using known techniques, such as PCR. In PCR, if the recovered nucleic acid is DNA, it can be used as it is, and if the recovered nucleic acid is mRNA, RT-PCR may be used to prepare cDNA, which is in turn used to amplify the nucleic acid.

Step (4') is a step of synthesizing a polypeptide including a cyclic peptide, a dimerization domain containing a cysteine residue and a leucine zipper region, and a peptide linker present between the cyclic peptide and the dimerization domain in a cell-free expression system using the nucleic acid amplified in step (3') to prepare a dimer of the polypeptide. This is as described herein above for the method of preparing dimerized cyclic peptides. The "polypeptide including a cyclic peptide, a dimerization domain containing a cysteine residue and a leucine zipper region, and a peptide linker present between the cyclic peptide and the dimerization domain" is as described herein above.

Step (5') is a step of measuring activity based on binding of the dimer to a target molecule.

In step (5'), the "activity based on binding of the dimer to a target molecule" refers to an action resulting from binding of the dimer to the target molecule and/or strength of the action, and can include both agonistic activity and antagonistic activity. For example, activity can include receptor activity, signaling activity, apoptotic activity, and the like. The "target molecule" in step (5') is as described herein above.

The measurement in step (5') can be carried out using known techniques, including bioluminescence assays such as luciferase reporter assay, receptor dimerization assays such as PathHunter^{®} Receptor Dimerization assay, or fluorescence resonance energy transfer (FRET) measurements, or cross-linking with chemical linkers and dimerization analysis by SDS-PAGE, or the like.

In an embodiment, in the method for screening dimerized cyclic peptides, step (1') includes creating a further library including a plurality of displayed polypeptides based on the obtained plurality of polypeptides, and repeating obtaining from the further library one or more polypeptides having binding affinity with the target molecule.

Here, based on the obtained plurality of polypeptides, the further library including a plurality of displayed polypeptides can be created by the following steps.

Step (1'-1): recovering a nucleic acid associated with the polypeptides obtained in step (1'), and

Step (1'-2): amplifying the nucleic acid recovered in step (1'-1).

Step (1'-1) can be carried out in the same manner as the method described as step (2'). Step (1'-2) can be carried out in the same manner as the method described as step (3'). Obtaining one or more polypeptides having binding affinity with the target molecule from the further library thus created can be carried out in the same manner as step (1').

In an embodiment, the method for screening dimerized cyclic peptides may include, in step (1'), creating a further library including a plurality of displayed polypeptides based on the obtained plurality of polypeptides, and repeating obtaining from the further library one or more polypeptides having binding affinity with the target molecule twice or more, three times or more, four times or more, five times or more, six times or more, or seven times or more.

An embodiment of the present invention relates to a library for screening dimerized cyclic peptides. Such a library, apart from the aforementioned library, includes, for example, a plurality of displayed polypeptides or a plurality of nucleic acids for expressing the plurality of displayed polypeptides, wherein the plurality of displayed polypeptides each include a cyclic peptide, a dimerization domain containing a cysteine residue and a leucine zipper region, and a peptide linker present between the cyclic peptide and the dimerization domain, and the plurality of displayed polypeptides include different cyclic peptides.

In this library, "cyclic peptides", "dimerization domain containing a cysteine residue and a leucine zipper region", and "peptide linker present between the cyclic peptide and the dimerization domain" of "plurality of displayed polypeptides" are as described herein above.

Such a library may include 2 or more, 8 or more, 16 or more, 32 or more, 48 or more, 96 or more, 200 or more, 1000 or more, 10000 or more, 10⁵ or more, 10⁶ or more, 10⁷ or more, 10⁸ or more, 10⁹ or more, or 10¹⁰ or more kinds of displayed polypeptides. Also, such a library can include 10¹⁵ or less, 10¹⁴ or less, or 10¹³ or less kinds of polypeptides or dimers of the polypeptides.

Alternatively, such a library may include 2 or more, 8 or more, 16 or more, 32 or more, 48 or more, 96 or more, 200 or more, 1000 or more, 10000 or more, 10⁵ or more, 10⁶ or more, 10⁷ or more, 10⁸ or more, 10⁹ or more, or 10¹⁰ or more kinds of nucleic acids for expressing a plurality of displayed polypeptides. Also, such a library can include 10¹⁵ or less, 10¹⁴ or less, or 10¹³ or less kinds of polypeptides or dimers of the polypeptides.

### Examples

The present invention will be described in more detail below with reference to Examples, but the present invention is not limited to these Examples.

All HGFs used in the Examples are recombinant human HGFs (rhHGF, R&D Systems), and in the present specification and drawings, recombinant human HGFs are simply referred to as HGFs.

### Example 1: Design of cyclic peptide-leucine zipper fusion and expression by cell-free expression system

### (1-1) Overview of design of aMD4dY-leucine zipper fusion

It has been reported that when dimerized, the cyclic peptide aMD4dY (SEQ ID NO: 2) exhibits an HGF-like activity, that is, acts as a Met agonist. Therefore, a peptide sequence was designed to express aMD4dY as a fusion with a leucine zipper region that self-dimerizes.

Also, the binding is reversible, as the leucine zipper region enables dimerization by non-covalent bonds. Therefore, a cysteine residue was added to form an intermolecular disulfide bond, thereby designing a peptide sequence that forms a tighter dimerization.

Specifically, the following peptide sequences were designed (Figs. 1 to 4).
· "aMD4dY" - "peptide linker" - "cysteine residue" - "leucine zipper region"
· "aMD4dY" - "peptide linker" - "leucine zipper region" - "cysteine residue"
· "leucine zipper region" - "cysteine residue" - "peptide linker" - "aMD4dY"
· "cysteine residue" - "leucine zipper region" - "peptide linker" - "aMD4dY"

Thus, it is possible to form a disulfide bond at the N-terminal side or C-terminal side of the leucine zipper region and covalently dimerize.

In addition, a construct with an N11 tag, which is an affinity tag, added to the N-terminus was also designed in the same manner. Specifically, the following peptide sequences were designed (Figs. 5 and 6).
· "tag sequence" - "leucine zipper region" - "cysteine residue" - "peptide linker" - "aMD4dY"
· "tag sequence" - "cysteine residue" - "leucine zipper region" - "peptide linker" - "aMD4dY"

The leucin zipper domains used were the leucine zipper domains of c-Jun, GCN4, and CEBPα proteins, which are known to form homodimers, and JunΔhep with deleted heparin-binding ability by substituting positively charged amino acids of c-Jun leucine zipper domain with glutamine (M. D. Ballinger et al., Nat. Biotechnol. 17, 1199-1204 (1999)). These leucine zipper domains are hereinafter referred to as Jun, GCN4, CEBPα, and JunΔhep, respectively.

### (1-2) Design of aMD4dY-leucine zipper fusion

The sequences of the designed aMD4dY-leucine zipper fusions are shown below. aMD4dY, Jun, GCN4, CEBPα, and JunΔhep are as described in (1-1) above, PA8 represents a peptide linker, and cys represents a cysteine residue.
(a) aMD4dY-PA8-cys-Jun 
MAETCRQFNRRTHEVWNLDCGAAPAAPAPAAGCGGRIARLEEKVKTLKAQNS
ELASTANMLREQVAQLKQKVMN (SEQ ID NO: 45)
(b) aMD4dY-PA8-Jun 
MAETCRQFNRRTHEVWNLDCGAAPAAPAPAAGGGRIARLEEKVKTLKAQNSEL
ASTANMLREQVAQLKQKVMN (SEQ ID NO: 46)
(c) aMD4dY-PA8-cys-JunΔhep 
MAETCRQFNRRTHEVWNLDCGAAPAAPAPAAGCGGQIAQLEEKVQTLQAQNS
ELASTANMLREQVAQLKQKVMN (SEQ ID NO: 47)
(d) aMD4dY-PA8-GCN4-cys 
MAETCRQFNRRTHEVWNLDCGAAPAAPAPGRMKQLEDKVEELLSKNYHLENE
VARLKKLVGERGGCGG (SEQ ID NO: 48)
(e) aMD4dY-PA8-cys-CEBPα 
MAETCRQFNRRTHEVWNLDCGAAPAAPAPAAGCGGRNVETQQKVLELTSDND
RLRKRVEQLSRELDTLRGIFRQ (SEQ ID NO: 49)
(f) cys-Jun-PA8-aMD4dY 
MGGCGGRIARLEEKVKTLKAQNSELASTANMLREQVAQLKQKVMNAAPAAPA
PACRQFNRRTHEVWNLDC (SEQ ID NO: 50)
(g) Jun-cys-PA8-aMD4dY 
MGRIARLEEKVKTLKAQNSELASTANMLREQVAQLKQKVMNGGCGGAAPAAP
APACRQFNRRTHEVWNLDC (SEQ ID NO: 51)
(h) N11-Jun-cys-PA8-aMD4dY 
MKDHLIHNHHKHEHAHAEHLGGRIARLEEKVKTLKAQNSELASTANMLREQV
AQLKQKVMNGGCGGAAPAAPAPACRQFNRRTHEVWNLDC (SEQ ID NO: 52)
(i) N11-cys-Jun-PA8-aMD4dY 
MKDHLIHNHHKHEHAHAEHLGGCGGRIARLEEKVKTLKAQNSELASTANMLR
EQVAQLKQKVMNAAPAAPAPACRQFNRRTHEVWNLDC (SEQ ID NO: 53)
(j) N11-GCN4-cys-PA8-aMD4dY 
MKDHLIHNHHKHEHAHAEHLGGRMKQLEDKVEELLSKNYHLENEVARLKKLV
GERGGCGGAAPAAPAPACRQFNRRTHEVWNLDC (SEQ ID NO: 54)
(k) N11-cys-GCN4-PA8-aMD4dY

MKDHLIHNHHKHEHAHAEHLGGCGGRMKQLEDKVEELLSKNYHLENEVARL
KKLVGERAAPAAPAPACRQFNRRTHEVWNLDC (SEQ ID NO: 55)

### (1-3) Expression of aMD4dY-leucine zipper fusion

The aMD4dY-leucine zipper fusion was subjected to expression studies using a cell-free expression system. Examples of expression studies are described below.

### (1-4) Construction of a gene construct of aMD4dY-leucine zipper fusion for cell-free expression

As nucleic acids for expressing the aMD4dY-leucine zipper fusion, nucleotide sequences encoding the above amino acid sequences (a) to (k) were designed. Furthermore, a T7 promoter sequence and a ribosome binding sequence necessary for cell-free expression were added to the 5'-side thereof, and a termination codon and an untranslated region were added to the 3'-side thereof. Nucleotide sequences for cell-free expression corresponding to the above (a) to (k) are shown in (l) to (v), respectively.
(l) Nucleotide sequence for expressing aMD4dY-PA8-cys-Jun 
GAAATTAATACGACTCACTATAGGGAGACCACAACGGTTTCCCTCTAGAAATA
ATTTTGTTTAACTTTAAGAAGGAGATATACCAATGGCAGAGACCTGTCGTCAG
TTTAACCGTCGTACGCACGAAGTTTGGAATCTGGACTGTGGCGCAGCCCCCG
CAGCACCAGCTCCAGCAGCAGGCTGCGGTGGTCGTATCGCTCGTCTGGAAG
AAAAAGTTAAAACCCTGAAAGCTCAGAACTCCGAACTGGCTTCCACCGCTA
ACATGCTGCGTGAACAGGTTGCTCAGCTGAAACAGAAAGTTATGAACTAGTG
AATAACTAATCC (SEQ ID NO: 56)
(m) Nucleotide sequence for expressing aMD4dY-PA8-Jun 
GAAATTAATACGACTCACTATAGGGAGACCACAACGGTTTCCCTCTAGAAATA
ATTTTGTTTAACTTTAAGAAGGAGATATACCAATGGCAGAGACCTGTCGTCAG
TTTAACCGTCGTACGCACGAAGTTTGGAATCTGGACTGTGGCGCAGCCCCCG
CAGCACCAGCTCCAGCAGCAGGCGGTGGTCGTATCGCTCGTCTGGAAGAAA
 
AAGTTAAAACCCTGAAAGCTCAGAACTCCGAACTGGCTTCCACCGCTAACAT
GCTGCGTGAACAGGTTGCTCAGCTGAAACAGAAAGTTATGAACTAGTGAATA
ACTAATCC (SEQ ID NO: 57)
(n) Nucleotide sequence for expressing aMD4dY-PA8-cys-JunΔhep 
GAAATTAATACGACTCACTATAGGGAGACCACAACGGTTTCCCTCTAGAAATA
ATTTTGTTTAACTTTAAGAAGGAGATATACCAATGGCAGAGACCTGTCGTCAG
TTTAACCGTCGTACGCACGAAGTTTGGAATCTGGACTGTGGCGCAGCCCCCG
CAGCACCAGCTCCAGCAGCAGGCTGCGGTGGTCAGATTGCCCAACTGGAAG
AGAAAGTGCAGACGTTACAAGCGCAAAACAGCGAACTGGCTTCGACCGCGA
ACATGTTGCGCGAACAGGTTGCACAGCTGAAACAGAAGGTCATGAATTAGTG
AATAACTAATCC (SEQ ID NO: 58)
(o) Nucleotide sequence for expressing aMD4dY-PA8-GCN4-cys 
GAAATTAATACGACTCACTATAGGGAGACCACAACGGTTTCCCTCTAGAAATA
ATTTTGTTTAACTTTAAGAAGGAGATATACCAATGGCAGAGACCTGTCGTCAG
TTTAACCGTCGTACGCACGAAGTTTGGAATCTGGACTGTGGCGCAGCACCCG
CAGCTCCAGCACCAGGCCGCATGAAACAGTTGGAGGATAAAGTCGAAGAGC
TTCTGAGCAAGAACTATCACCTCGAAAACGAAGTTGCTCGTCTGAAGAAACT
CGTTGGAGAACGTGGTGGTTGTGGCGGGTAGTGAATAACTAATCC (SEQ ID
NO: 59)
(p) Nucleotide sequence for expressing aMD4dY-PA8-cys-CEBPα 
GAAATTAATACGACTCACTATAGGGAGACCACAACGGTTTCCCTCTAGAAATA
ATTTTGTTTAACTTTAAGAAGGAGATATACCAATGGCAGAGACCTGTCGTCAG
TTTAACCGTCGTACGCACGAAGTTTGGAATCTGGACTGTGGCGCAGCCCCCG
CAGCACCAGCTCCAGCAGCAGGCTGCGGTGGTCGCAATGTGGAGACTCAGC
AGAAGGTCCTGGAACTGACGTCTGACAACGATCGGTTACGCAAACGGGTTG
AACAGCTGTCTCGTGAACTCGACACTCTTCGTGGCATCTTCCGCCAATAGTG
AATAACTAATCC (SEQ ID NO: 60)
(q) Nucleotide sequence for expressing cys-Jun-PA8-aMD4dY 
GAAATTAATACGACTCACTATAGGGAGACCACAACGGTTTCCCTCTAGAAATA
ATTTTGTTTAACTTTAAGAAGGAGATATACCAATGGGTGGTTGCGGTGGTCGT
 
ATCGCTCGTCTGGAAGAAAAAGTTAAAACCCTGAAAGCTCAGAACTCCGAA
CTGGCTTCCACCGCTAACATGCTGCGTGAACAGGTTGCTCAGCTGAAACAGA
AAGTTATGAACGCAGCCCCCGCAGCACCAGCTCCAGCATGTCGTCAGTTTAA
CCGTCGTACGCACGAAGTTTGGAATCTGGACTGTTAGTGAATAACTAATCC
(SEQ ID NO: 61)
(r) Nucleotide sequence for expressing Jun-cys-PA8-aMD4dY 
GAAATTAATACGACTCACTATAGGGAGACCACAACGGTTTCCCTCTAGAAATA
ATTTTGTTTAACTTTAAGAAGGAGATATACCAATGGGTCGTATCGCTCGTCTG
GAAGAAAAAGTTAAAACCCTGAAAGCTCAGAACTCCGAACTGGCTTCCACC
GCTAACATGCTGCGTGAACAGGTTGCTCAGCTGAAACAGAAAGTTATGAACG
GTGGTTGCGGTGGTGCAGCCCCCGCAGCACCAGCTCCAGCATGTCGTCAGTT
TAACCGTCGTACGCACGAAGTTTGGAATCTGGACTGTTAGTGAATAACTAATC
C (SEQ ID NO: 62)
(s) Nucleotide sequence for expressing N11-Jun-cys-PA8-aMD4dY 
GAAATTAATACGACTCACTATAGGGAGACCACAACGGTTTCCCTCTAGAAATA
ATTTTGTTTAACTTTAAGAAGGAGATATACCAATGAAAGATCATCTCATCCAC
AATCATCACAAACATGAGCACGCTCATGCCGAACACCTGGGTGGTCGTATCG
CTCGTCTGGAAGAAAAAGTTAAAACCCTGAAAGCTCAGAACTCCGAACTGG
CTTCCACCGCTAACATGCTGCGTGAACAGGTTGCTCAGCTGAAACAGAAAGT
TATGAACGGTGGTTGCGGTGGTGCAGCACCAGCAGCTCCGGCCCCTGCTTGT
CGTCAGTTTAACCGTCGTACGCACGAAGTTTGGAATCTGGACTGTTAGTGAAT
AACTAATCC (SEQ ID NO: 63)
(t) Nucleotide sequence for expressing N11-cys-Jun-PA8-aMD4dY 
GAAATTAATACGACTCACTATAGGGAGACCACAACGGTTTCCCTCTAGAAATA
ATTTTGTTTAACTTTAAGAAGGAGATATACCAATGAAAGATCATCTCATCCAC
AATCATCACAAACATGAGCACGCTCATGCCGAACACCTGGGTGGTTGCGGTG
GTCGTATCGCTCGTCTGGAAGAAAAAGTTAAAACCCTGAAAGCTCAGAACTC
CGAACTGGCTTCCACCGCTAACATGCTGCGTGAACAGGTTGCTCAGCTGAAA
CAGAAAGTTATGAACGCAGCACCAGCAGCTCCGGCCCCTGCTTGTCGTCAGT
TTAACCGTCGTACGCACGAAGTTTGGAATCTGGACTGTTAGTGAATAACTAAT
 CC (SEQ ID NO: 64)
(u) Nucleotide sequence for expressing N11-GCN4-cys-PA8-aMD4dY 
GAAATTAATACGACTCACTATAGGGAGACCACAACGGTTTCCCTCTAGAAATA
ATTTTGTTTAACTTTAAGAAGGAGATATACCAATGAAAGATCATCTCATCCAC
AATCATCACAAACATGAGCACGCTCATGCCGAACACCTGGGTGGTCGCATGA
AACAGTTGGAGGATAAAGTCGAAGAGCTTCTGAGCAAGAACTATCACCTCG
AAAACGAAGTTGCTCGTCTGAAGAAACTCGTTGGAGAACGTGGTGGTTGCG
GTGGTGCAGCACCAGCAGCTCCGGCCCCTGCTTGTCGTCAGTTTAACCGTCG
TACGCACGAAGTTTGGAATCTGGACTGTTAGTGAATAACTAATCC (SEQ ID
NO: 65)
(v) Nucleotide sequence for expressing N11-cys-GCN4-PA8-aMD4dY

GAAATTAATACGACTCACTATAGGGAGACCACAACGGTTTCCCTCTAGAAATA
ATTTTGTTTAACTTTAAGAAGGAGATATACCAATGAAAGATCATCTCATCCAC
AATCATCACAAACATGAGCACGCTCATGCCGAACACCTGGGTGGTTGCGGTG
GTCGCATGAAACAGTTGGAGGATAAAGTCGAAGAGCTTCTGAGCAAGAACT
ATCACCTCGAAAACGAAGTTGCTCGTCTGAAGAAACTCGTTGGAGAACGTG
CAGCACCAGCAGCTCCGGCCCCTGCTTGTCGTCAGTTTAACCGTCGTACGCA
CGAAGTTTGGAATCTGGACTGTTAGTGAATAACTAATCC (SEQ ID NO: 66)

The DNA sequences shown in (l) to (v) were totally synthesized and cloned into pEX-K4J2 plasmid vectors at Eurofins Genomics K.K.

### (1-5) Overview of design of EMP-leucine zipper fusion

EMP, a cyclic peptide described in Johnson, D. L. et al. Chem. Biol. 1997, 4, 939-950, has been reported to exhibit potent erythropoietin (EPO)-like activity upon dimerization. Therefore, a peptide sequence was designed to express EMP as a fusion with a leucine zipper domain that self-dimerizes. In addition, a cysteine residue was added in the same manner as in (1-1) above, and the peptide sequence was designed to be "EMP" - "peptide linker" - "cysteine residue" - "leucine zipper region".

### (1-6) Design of EMP-leucine zipper fusion

The sequence of the designed aMD4dY-leucine zipper fusion is shown below. (w) EMP-PA8-cys-Jun

MGGLYACHMGPMTWVCQPLRGAAPAAPAPAAGCGGRIARLEEKVKTLKAQNS
ELASTANMLREQVAQLKQKVMN (SEQ ID NO: 67)

EMP is as described above (1-5), PA8 represents a peptide linker, cys represents a cysteine residue, and Jun represents the leucine zipper domain of the c-Jun protein.

### (1-7) Expression of EMP-leucine zipper fusion

The EMP-leucine zipper fusion was subjected to expression studies using a cell-free expression system. Examples of expression studies are described below.

### (1-8) Construction of a gene construct of EMP-leucine zipper fusion for cell-free expression

As a nucleic acid for expressing the EMP-leucine zipper fusion, a nucleotide sequence encoding the above amino acid sequence (w) was designed. Furthermore, a T7 promoter sequence and a ribosome binding sequence necessary for cell-free expression were added to the 5'-side thereof, and a termination codon and an untranslated region were added to the 3'-side thereof. A nucleotide sequence for cell-free expression corresponding to the above (w) is shown in (x).
(x) Nucleotide sequence for expressing EMP-PA8-cys-Jun

GAAATTAATACGACTCACTATAGGGAGACCACAACGGTTTCCCTCTAGAAATA
ATTTTGTTTAACTTTAAGAAGGAGATATACCAATGGGTGGCCTGTATGCGTGC
CATATGGGGCCAATGACCTGGGTGTGTCAGCCGTTACGCGGCGCAGCCCCCG
CAGCACCAGCTCCAGCAGCAGGCTGCGGTGGTCGTATCGCTCGTCTGGAAG
AAAAAGTTAAAACCCTGAAAGCTCAGAACTCCGAACTGGCTTCCACCGCTA
ACATGCTGCGTGAACAGGTTGCTCAGCTGAAACAGAAAGTTATGAACTAGTG
AATAACTAATCC (SEQ ID NO: 68)

The DNA sequence shown in (x) was totally synthesized and cloned into a pEX-K4J2 plasmid vector at Eurofins Genomics K.K.

### (1-9) Cell-free expression of cyclic peptide-leucine zipper fusion

The genes of the cyclic peptide-leucine zipper fusions constructed above were amplified by PCR. PCR was carried out using primers having the sequences set forth in (y) and (z) below and KOD -Plus- Ver. 2 (Toyobo) as a PCR enzyme.
(y) Cell-free primer Fwd:
   GAAATTAATACGACTCACTATAGGG (SEQ ID NO: 69)
(z) Cell-free primer Rev:
   CACTGACTGGATTAGTTATTCAC (SEQ ID NO: 70)

Next, based on the gene fragments amplified by PCR, cyclic peptide-leucine zipper fusions were expressed in a cell-free expression system. PUREfrex 2.0 (Gene Frontier) or PURExpress In Vitro Protein Synthesis Kit (New England BioLabs) was used for cell-free expression.

For cell-free expression with PUREfrex 2.0, to 4 ng of the DNA sequence fragments of any of (l) to (v) or (x), or to 90 ng of each plasmid vector containing the DNA sequence of any of (l) to (v) or (x), 10 µL of Solution I, 1 µL of Solution II, 2 µL of Solution III, and 7 µL of water were added, and the mixture was incubated at 37°C for 6 hours to carry out translation reaction.

For cell-free expression with PURExpress In Vitro Protein Synthesis Kit, to 90 ng of each plasmid vector containing the DNA sequence of any of (l) to (v) or (x), 8 µL of Solution A, 6 µL of Solution B, and 6 µL of water were added, and the mixture was incubated at 37°C for 6 hours to carry out translation reaction.

### (1-10) Confirmation of expression of cyclic peptide-leucine zipper fusion by SDS-PAGE

The reaction solution containing each cyclic peptide-leucine zipper fusion expressed cell-free in (1-9) above, in an amount of 3 µL, was subjected to SDS-PAGE using NuPAGE^{®} 12% Bis-Tirs Gel (Thermo Fisher Scientific) and then stained with Quick-CBB (Wako). Fig. 7 shows the results of using each reaction solution for (i) N11-cys-Jun-PA8-aMD4dY or (k) N11-cys-GCN4-PA8-aMD4dY, which were expressed cell-free using PUREfrex 2.0, and the results of subjecting each reaction solution to PAGE after purification with Dynabeads His-Tag Isolation & Pulldown. Protein bands presumed to be the respective aMD4dY-leucine zipper fusions (Monomer) and their disulfide dimers (Dimer) were detected in the lanes subjected to N11 tag purification.

### Example 2: Activity evaluation of aMD4dY-leucine zipper fusion

The dimer of each aMD4dY-leucine zipper fusion was evaluated by luciferase assay. To 25 µL of Opti-MEM medium (Thermo Fisher Scientific), 0.6 µL of Attractene Transfection Reagent (QIAGEN) was added and incubated at room temperature for 5 minutes. To the above mixed solution, a mixed solution of 25 µL of Opti-MEM and 1 µL of SRE reporter vector (QIAGEN) was added and incubated at room temperature for 20 minutes. This mixed solution was added to a 96-well plate, HEK293E cells were seeded thereon at 40,000 cells/well, and incubated overnight at 37°C in a 5% CO₂ incubator. After transfection, all the culture supernatant was removed, and 100 µL of Opti-MEM medium (evaluation basal medium), containing 0.5% FBS (Thermo Fisher Scientific), 1% non-essential amino acid solution (Thermo Fisher Scientific), and penicillin-streptomycin (Nacalai Tesque), was added thereto, which was cultured at 37°C for 4 hours to starve the cells.

To the evaluation basal medium, 0 to 100 ng/mL HGF was added, or the cell-free expression reaction solution was added at a 25- to 625-fold dilution with respect to the medium, and 100 µL of the evaluation basal medium was added to the cells. The cells were stimulated by culturing overnight at 37°C in a 5% CO₂ incubator.

Dual-Luciferase Reporter Assay System (Promega) was used for signal intensity detection. The medium supernatant in an amount of 100 µL was removed, 50 µL of Glo reagent was added, and cells were lysed at room temperature for 10 minutes. Activation of the HGF-Erk-SRE pathway was quantified by detecting Firefly luciferase luminescence from cell lysates with a plate reader. Subsequently, 50 µL of Glo & Stop reagent solution was added, and after 10 minutes, luminescence of Renilla luciferase, an internal standard, was detected to quantify the cell count. The signal activity of each sample was quantified as SRE activity = (luminescence intensity of Firefly luciferase)/(luminescence intensity of Renilla luciferase). A signal activity value relative to a sample without an addition of evaluation compound was obtained and defined as relative reporter activity.

As shown in Fig. 8, when expressed, aMD4dY-PA8-cys-Jun showed activity comparable to HGF, but when expressed, aMD4dY-PA8-Jun did not show activity. From this, it became clear that tight dimerization by disulfide bonds formed by cysteine is necessary for the activity of cyclic peptides (growth factor-like activity) to be exhibited.

As shown in Fig. 9, activity was also exhibited in the case of expressing aMD4dY-PA8-cys-JunΔhep having its heparin binding ability removed. This revealed that the heparin binding ability of the leucine zipper region was not required for the activity of cyclic peptides (growth factor-like activity).

As shown in Figs. 10 and 11, when aMD4dY-PA8-GCN4-cys and aMD4dY-PA8-cys-CEBPα were expressed, each dimer was found to have signal activation ability. From this, it was understood that it is possible to widely use the leucine zipper region that forms homodimers in the dimerization domain.

As shown in Fig. 12, when cys-Jun-PA8-aMD4dY and Jun-cys-PA8-aMD4dY were expressed, each dimer was found to have signal activation ability. From this, it was understood that the activity of cyclic peptides (growth factor-like activity) can be obtained even if the order of the cyclic peptide, peptide linker, cysteine residue, and leucine zipper region is changed.

As shown in Fig. 13, it was revealed that even when the construct with the N11 tag added to the N-terminus was used, the ability to activate signals was exhibited. From this, it was understood that it was also possible to add a tag sequence to the N-terminus of an expressed polypeptide.

### Example 3: Activity evaluation of EMP-leucine zipper fusion

The ability of the EMP-leucine zipper fusion dimer to activate EpoR was assessed using PathHunter^{®} eXpress EpoR-JAK2 Functional Assay Kit (DiscoveRx).

Cells for EPO activity evaluation contained in the PathHunter^{®} eXpress EpoR-JAK2 Functional Assay Kit were seeded in a 96-well plate and cultured for 24 hours at 37°C under 5% CO₂. A cell-free expression reaction solution containing the EMP-leucine zipper fusion were diluted and added to the cells. As a negative control, an unreacted solution diluted at the same factor as this reaction solution and subjected to treatment for cell-free expression in the same manner as described in (1-9) above, except that the cell-free expression system did not contain the gene of the sequence represented by (x). To the cells stimulated at room temperature for 3 hours, the prepared Substrate Reagent was added and incubated for 60 minutes. Chemiluminescence intensity was quantified with a Nivo plate reader (Perkin Elmer).

The results are shown in Fig. 14. These results revealed that the EMP-PA8-cys-Jun dimer activated EpoR.

### Example 4: Screening to enrich HGF-like active molecules from a cyclic peptide-leucine zipper fusion library based on binding to c-Met molecules

The fusions were expressed by cell-free translation from a plurality of DNAs each encoding a cyclic peptide-leucine zipper fusion molecule, fusion molecules that bind c-Met and the DNA encoding them were enriched, and the enriched fusion molecules were confirmed to have HGF-like activity. In this Example, the ribosome display method and the cyclic peptide-leucine zipper fusion were combined to isolate the complex of the cyclic peptide-leucine zipper fusion that binds to c-Met immobilized on magnetic beads, a ribosome, and a mRNA complex. Through a series of operations, it was confirmed that template DNA for cyclic peptide-leucine zipper fusions that bind c-Met were enriched from the library, and that cell-free translation products of the enriched DNA had HGF-like activity that activates c-Met.

### (4-1) Design of cyclic peptide-leucine zipper fusion subjected to ribosome display method

The sequences of the designed cyclic peptide-leucine zipper fusions are shown below. Each fusion was designed to be "target receptor binding cyclic peptide" - "peptide linker" - "leucine zipper" - "cysteine" - "N11 tag" - "SecM sequence". As the target receptor binding cyclic peptides, c-Met binding cyclic peptide (aMD4dY) or thrombopoietin receptor binding cyclic peptide (TPO) or erythropoietin receptor binding cyclic peptide (EMP') was selected. The SecM sequence (referred to as SecM below) is a sequence represented by the amino acid sequence of FSTPVWISQAQGIRAG (SEQ ID NO: 86), and aims to form a ribosome-cyclic peptide-leucine zipper fusion-mRNA complex by inhibiting the elongation reaction of the ribosome. The sequence denoted as GS3 in the following is a sequence composed of 3 repeats of "glycine" - "serine" and is a spacer sequence.
(A) aMD4dY-Jun-cys-N11-SecM-GS3 
MAETCRQFNRRTHEVWNLDCGAAPAAPAPAAGGRIARLEEKVKTLKAQNSELA
STANMLREQVAQLKQKVMNGCGGKDHLIHNHHKHEHAHAEHLFSTPVWISQA
QGIRAGGSGSGS (SEQ ID NO: 71)
(B) TPO-Jun-cys-N11-SecM-GS3 
MAETCADGPTLREWISFCGAAPAAPAPAAGGRIARLEEKVKTLKAQNSELASTA
NMLREQVAQLKQKVMNGCGGKDHLIHNHHKHEHAHAEHLFSTPVWISQAQGI
RAGGSGSGS (SEQ ID NO: 72)
(C) EMP'-Jun-cys-N11-SecM-GS3

MAETCHMGPMTWVCGAAPAAPAPAAGGRIARLEEKVKTLKAQNSELASTANM
LREQVAQLKQKVMNGCGGKDHLIHNHHKHEHAHAEHLFSTPVWISQAQGIRA
GGSGSGS (SEQ ID NO: 73)

### (4-2) Construction of template DNA for cyclic peptide-leucine zipper fusion

The nucleotide sequences encoding the above amino acid sequences (A) to (C) were designed as template DNAs for expressing the cyclic peptide-leucine zipper fusions. Furthermore, a T7 promoter sequence and a ribosome binding sequence necessary for cell-free expression were added to the 5'-side thereof, and a termination codon and an untranslated region were added to the 3'-side thereof to design (D) to (F). In addition, single-stranded DNA (G) to (O) was designed to prepare these template DNAs by PCR.

The single-stranded DNA sequences shown in (G) to (O) were totally synthesized at Eurofins Genomics K.K.
(D) Nucleotide sequence for expressing aMD4dY-Jun-cys-N11-SecM-GS3 
GAAATTAATACGACTCACTATAGGGAGACCACAACGGTTTCCCTCTAGAAATA
ATTTTGTTTAACTTTAAGAAGGAGATATACCAATGGCAGAGACCTGTCGTCAG
TTTAACCGTCGTACGCACGAAGTTTGGAATCTGGACTGTGGCGCAGCTCCCG
CAGCACCAGCTCCAGCAGCAGGCGGTCGTATCGCTCGTCTGGAAGAAAAAG
TTAAAACCCTGAAAGCTCAGAACTCCGAACTGGCTTCCACCGCTAACATGCT
 
GCGTGAACAGGTTGCTCAGCTGAAACAGAAAGTTATGAACGGCTGCGGTGG
CAAAGATCATCTCATCCACAATCATCACAAACATGAGCACGCTCATGCCGAA
CACCTGTTTTCTACTCCTGTTTGGATTTCTCAAGCTCAAGGTATTCGTGCTGG
TGGTAGCGGTAGCGGTAGCTAGTGAATAACTAATCC (SEQ ID NO: 74)
(E) Nucleotide sequence for expressing TPO-Jun-cys-N11-SecM-GS3 
GAAATTAATACGACTCACTATAGGGAGACCACAACGGTTTCCCTCTAGAAATA
ATTTTGTTTAACTTTAAGAAGGAGATATACCAATGGCAGAGACCTGTGCGGAC
GGTCCGACGTTACGCGAATGGATCAGCTTTTGTGGCGCAGCTCCCGCAGCAC
CAGCTCCAGCAGCAGGCGGTCGTATCGCTCGTCTGGAAGAAAAAGTTAAAA
CCCTGAAAGCTCAGAACTCCGAACTGGCTTCCACCGCTAACATGCTGCGTGA
ACAGGTTGCTCAGCTGAAACAGAAAGTTATGAACGGCTGCGGTGGCAAAGA
TCATCTCATCCACAATCATCACAAACATGAGCACGCTCATGCCGAACACCTGT
TTTCTACTCCTGTTTGGATTTCTCAAGCTCAAGGTATTCGTGCTGGTGGTAGC
GGTAGCGGTAGCTAGTGAATAACTAATCC (SEQ ID NO: 75)
(F) Nucleotide sequence for expressing EMP'-Jun-cys-N11-SecM-GS3 
GAAATTAATACGACTCACTATAGGGAGACCACAACGGTTTCCCTCTAGAAATA
ATTTTGTTTAACTTTAAGAAGGAGATATACCAATGGCAGAGACCTGTCATATG
GGGCCAATGACCTGGGTGTGTGGCGCAGCTCCCGCAGCACCAGCTCCAGCA
GCAGGCGGTCGTATCGCTCGTCTGGAAGAAAAAGTTAAAACCCTGAAAGCT
CAGAACTCCGAACTGGCTTCCACCGCTAACATGCTGCGTGAACAGGTTGCTC
AGCTGAAACAGAAAGTTATGAACGGCTGCGGTGGCAAAGATCATCTCATCCA
CAATCATCACAAACATGAGCACGCTCATGCCGAACACCTGTTTTCTACTCCTG
TTTGGATTTCTCAAGCTCAAGGTATTCGTGCTGGTGGTAGCGGTAGCGGTAGC
TAGTGAATAACTAATCC (SEQ ID NO: 76)
(G) Nucleotide sequence 1 of single-stranded DNA for preparing (D) to (F) 
CAATGGCAGAGACCTGTCGTCAGTTTAACCGTCGTACGCACGAAGTTTGGAA
TCTGGACTGTGGCGCAGCTCCCGC (SEQ ID NO: 77)
(H) Nucleotide sequence 2 of single-stranded DNA for preparing (D) to (F) 
CAATGGCAGAGACCTGTGCGGACGGTCCGACGTTACGCGAATGGATCAGCTT
TTGTGGCGCAGCTCCCGC (SEQ ID NO: 78)
(I) Nucleotide sequence 3 of single-stranded DNA for preparing (D) to (F) 
CAATGGCAGAGACCTGTCATATGGGGCCAATGACCTGGGTGTGTGGCGCAGC
TCCCGC (SEQ ID NO: 79)
(J) Nucleotide sequence 4 of single-stranded DNA for preparing (D) to (F) 
GAAATTAATACGACTCACTATAGGGAGACCACAACGGTTTCCCTCTAGAAATA
ATTTTGTTTAACTTTAAGAAGGAGATATACCAATGGCAGAGACCTGT (SEQ ID
NO: 80)
(K) Nucleotide sequence 5 of single-stranded DNA for preparing (D) to (F) 
TGTGGCGCAGCTCCCGCAGCACCAGCTCCAGCAGCAGGCGGTCGTATCGCTC
GTCTGGAAGAAAAAGTTAAAACCCTGAAAGCTCAGAACTCCGAACTGGCTT
CCACCGCTAACATGCTGCGTGAACAGGTTGCTCAGCTGAAACAGAAAGTTAT
GAAC (SEQ ID NO: 81)
(L) Nucleotide sequence 6 of single-stranded DNA for preparing (D) to (F) 
ACCAGCACGAATACCTTGAGCTTGAGAAATCCAAACAGGAGTAGAAAACAG
GTGTTCGGCATGAGCGTGCTCATGTTTGTGATGATTGTGGATGAGATGATCTT
TGCCACCGCAGCCGTTCATAACTTTCTGTTTCAGC (SEQ ID NO: 82)
(M) Nucleotide sequence 7 of single-stranded DNA for preparing (D) to (F) 
GGATTAGTTATTCACTAGCTACCGCTACCGCTACCACCAGCACGAATACCTTG
(SEQ ID NO: 83)
(N) Nucleotide sequence 8 of single-stranded DNA for preparing (D) to (F) (selec_PCR_F1.F39)
   GAAATTAATACGACTCACTATAGGGAGACCACAACGGTT (SEQ ID NO: 84)
(O) Nucleotide sequence 9 of single-stranded DNA for preparing (D) to (F) (selec_PCR_R2.R24)
   GGATTAGTTATTCACTAGCTACCG (SEQ ID NO: 85)

### (4-3) Preparation of library

Three types of DNA sequences were prepared by PCR, aMD4dY-Jun-cys-N11-SecM-GS3, TPO-Jun-cys-N11-SecM-GS3, and EMP'-Jun-cys-N11-SecM-GS3. A DNA mix was prepared by mixing DNAs, aMD4dY-Jun-cys-N11-SecM-GS3, TPO-Jun-cys-N11-SecM-GS3, and EMP'-Jun-cys-N11-SecM-GS3, at a ratio of 1:50:50.

### (4-4) Preparation of mRNA library by transcription reaction

DNA transcription was carried out using T7 RNA polymerase. T7 RNA polymerase (Takara Bio, 2540A) and rNTPs (Promega, E6000) were used for transcription. 11 µL of ultrapure water, 2 µL of 10 x T7 RNA polymerase buffer, 2 µL of 50 mM DTT, 2 µL of 25 mM rNTPs, 1 µL of T7 RNA polymerase, and 1 µL of DNA mix were mixed and reacted in a constant temperature bath at 37°C for 6 hours. A 50 mM EDTA solution was then added. It was purified with RNA Clean & Concentrator 5 (ZYMO RESEARCH, R1015) and made into a 2 µM mRNA mix.

### (4-5) Translation of mRNA library and formation of ribosome-cyclic peptide-leucine zipper fusion-mRNA complex

PURExpress ΔRF1,2,3 kit was used for translation expression of the prepared library. 2 µL of solution A, 1.5 µL of solution B, 1 µL of mRNA mix, and 0.5 µL of ultrapure water were mixed and cultured in a 37°C constant temperature bath for 2 hours. After the reaction, the reaction vessel was placed on ice, and 20 µL of ice-cold 1 x TBSM-T buffer (50 mM Tris-HCl, 150 mM NaCl, 50 mM MgCl₂, 0.05% Tween 20) was added to the formed ribosome-cyclic peptide-leucine zipper fusion-mRNA complex. This complex reaction solution was used in (4-7) below.

### (4-6) Immobilization of target molecule

Immobilization of target molecules to magnetic beads was carried out. 5 µL of 2 pM Recombinant Human HGFR/c-MET Fc Chimera Protein (R&D systems, 8614-MT-100), 5 µL slurry of Dynabeads (invitrogen, DB10003), and 10 µL of 1 × TBS-T buffer (50 mM Tris-HCl, 150 mM NaCl, 0.05% Tween 20) were mixed and the mixture was subjected to inversion mixing for 30 minutes at 4°C. The magnetic beads were washed three times with 100 µL of 1 x TBS-T buffer, then resuspended in 10 µL of 1 x TBS-T buffer and allowed to stand on ice.

### (4-7) Mixing and isolation of ribosome-cyclic peptide-leucine zipper fusion-mRNA complex and target molecule

From the magnetic bead suspension, 1 x TBS-T buffer was removed, and the entire amount of the complex reaction solution in (4-5) above was added thereto. After slow resuspension, the mixture was subjected to inversion mixing at 4°C for 30 minutes. The magnetic bead suspension was washed three times with 200 µL of ice-cold 1 x TBSM-T buffer. To magnetic beads isolated using a neodymium magnet, 20 µL of 1 x TBS-T buffer and 5 µL of 500 mM EDTA aqueous solution were added, mixed well, and allowed to stand at room temperature for 5 minutes to release mRNA from the ribosome-cyclic peptide-leucine zipper fusion-mRNA complex.

### (4-8) Purification of mRNA

Using RNA Clean & Concentrator 5, mRNA was purified from the recovered solution.

### (4-9) Reverse transcription of purified mRNA

The purified mRNA was reverse transcribed with SuperScript^{™} II Reverse Transcriptase (Invitrogen, 18064022). 1 µL of the purified mRNA solution, 0.5 µL of 100 µM selec_PCR_R2.R24, 2.5 µL of dNTPs, and 2 µL of ultrapure water were mixed and heated at 70°C for 5 minutes. The mixture was cooled slowly at room temperature and centrifuged in a tabletop centrifuge. 2 µL of 5 x First-strand buffer, 1 µL of 0.1 M DTT solution, 0.5 µL of ultrapure water, and 0.5 µL of SuperScript II Reverse Transcriptase were added in order, which was reacted at 42°C for 50 minutes to obtain an mRNA-cDNA complex.

### (4-10) PCR amplification of cDNA

The recovered cDNA was amplified by PCR. KOD -Plus- Ver. 2 (Toyobo, KOD-211) was used for PCR. To 4 µL of the completed reverse transcription reaction, 10 µL of 10 x PCR buffer, 4 µL of 250 mM MgSO₄, 10 µL of 2 mM dNTPs, 1 µL of 100 µM selec_PCR_F1.F39, 1 µL of 100 µM selec_PCR_R2.R24, 63 µL of ultrapure water, and 2 µL of KOD -Plus- were mixed. A cycle was created under the conditions of 94°C for 30 seconds, 55°C for 40 seconds, and 68°C for 60 seconds, and was repeated 20 to 25 times. The recovered DNA was used as DNA mix2.

### (4-11) DNA length analysis of cDNA

Three types of DNAs, aMD4dY-Jun-cys-N11-SecM-GS3, TPO-Jun-cys-N11-SecM-GS3, and EMP'-Jun-cys-N11-SecM-GS3, along with DNA mix and DNA mix2 were evaluated with 2100 Bioanalyzer (Agilent technologies). Fig. 15 shows the results. Lanes 1 to 5 correspond to aMD4dY-Jun-cys-N11-SecM-GS3, TPO-Jun-cys-N11-SecM-GS3, EMP'-Jun-cys-N11-SecM-GS3, DNA mix, and DNA mix2 in order, respectively. When the band of DNA mix2 obtained after carrying out the ribosome display method was compared with the band of DNA mix, a shift in the direction of increasing DNA length was confirmed, and in DNA mix2, the band showing the same DNA length as the DNA encoding aMD4dY-Jun-cys-N11-SecM-GS3 showed the highest intensity.

### (4-12) Activity evaluation of cell-free translation product from amplified cDNA

Three types of DNAs, aMD4dY-Jun-cys-N11-SecM-GS3, TPO-Jun-cys-N11-SecM-GS3, and EMP'-Jun-cys-N11-SecM-GS3, along with DNA mix and DNA mix2 were used to carry out cell-free translation with PUREfrex 2.0, and each cell-free translation product was evaluated by luciferase assay activity. For cell-free translation, to 20 ng of each DNA, 2.5 µL of Solution I, 0.25 µL of Solution II, 0.5 µL of Solution III, and 1.75 µL of water were added, and the mixture was incubated at 37°C for 6 hours to carry out the translation reaction.

Reporter assays were carried out as follows. To 25 µL of Opti-MEM medium (Thermo Fisher Scientific), 0.6 µL of Attractene Transfection Reagent (QIAGEN) was added and incubated at room temperature for 5 minutes. To the above mixed solution, a mixed solution of 25 µL of Opti-MEM and 1 µL of SRE reporter vector (QIAGEN) was added and incubated at room temperature for 20 minutes. This mixed solution was added to a 96-well plate, HEK293E cells were seeded thereon at 40,000 cells/well, and incubated overnight at 37°C in a 5% CO₂ incubator. After transfection, all the culture supernatant was removed, and 100 µL of Opti-MEM medium (evaluation basal medium), containing 0.5% FBS (Thermo Fisher Scientific), 1% non-essential amino acid solution (Thermo Fisher Scientific), and penicillin-streptomycin (Nacalai Tesque), was added thereto, which was cultured at 37°C for 4 hours to starve the cells.

To the evaluation basal medium, 0 to 100 ng/mL HGF was added, or the above cell-free expression reaction solution was added at a 25- to 625-fold dilution with respect to the medium, and 100 µL of the evaluation basal medium was added to the cells. The cells were stimulated by culturing overnight at 37°C in a 5% CO₂ incubator.

Dual-Luciferase Reporter Assay System (Promega) was used for signal intensity detection. The medium supernatant in an amount of 100 µL was removed, 50 µL of Glo reagent was added, and cells were lysed at room temperature for 10 minutes. Activation of the HGF-Erk-SRE pathway was quantified by detecting Firefly luciferase luminescence from cell lysates with a plate reader. Subsequently, 50 µL of Glo & Stop reagent solution was added, and after 10 minutes, luminescence of Renilla luciferase, an internal standard, was detected to quantify the cell count. The signal activity of each sample was quantified as SRE activity = (luminescence intensity of Firefly luciferase)/(luminescence intensity of Renilla luciferase). A signal activity value relative to a sample without an addition of evaluation compound was obtained and defined as relative SRE reporter activity.

Fig. 16 shows the results. It has been revealed that DNA mix shows no relative receptor activity, whereas DNA mix2 shows similar activity to HGF. In addition, among the cell-free translation products of aMD4dY-Jun-cys-N11-SecM-GS3, TPO-Jun-cys-N11-SecM-GS3, and EMP'-Jun-cys-N11-SecM-GS3, only aMD4dY-Jun-cys-N11-SecM-GS3 showed activity, so that it has been found that DNA mix2 is enriched with aMD4dY-Jun-cys-N11-SecM-GS3. Based on the above results, it has been revealed that this approach makes it possible to obtain, from a library including a plurality of displayed cyclic peptide-leucine zipper fusions, a cyclic peptide-leucine zipper fusion that has binding affinity with the target molecule and activity resulting from the binding.

### Example 5: Screening method for enrichment of active molecules from a cyclic peptide-leucine zipper fusion library based on binding to EPO receptor molecules

In order to demonstrate that the screening method carried out in Example 4 is possible regardless of a target molecule, peptides that bind to the EPO receptor were enriched from a plurality of cyclic peptide-leucine zipper fusion molecules, and the presence or absence of activity based on the binding of the enriched DNA to the EPO receptor was confirmed. In this Example, the ribosome display method and the cyclic peptide-leucine zipper fusion were combined to recover the complex of the cyclic peptide-leucine zipper fusion that binds to the EPO receptor immobilized on magnetic beads, a ribosome, and a mRNA complex. Through a series of operations, it was confirmed that template DNAs for cyclic peptide-leucine zipper fusions that bind to the EPO receptor were enriched from the library.

### (5-1) Design of cyclic peptide-leucine zipper fusion for ribosome display method

The sequence of the newly designed cyclic peptide-leucine zipper fusion in addition to Example 4 is shown below.
(i) EMP-Jun-cys-N11-SecM-GS3

MAETGGLYACHMGPMTWVCQPLRGAAPAAPAPAAGGRIARLEEKVKTLKAQN
SELASTANMLREQVAQLKQKVMNGCGGKDHLIHNHHKHEHAHAEHLFSTPVW
ISQAQGIRAGGSGSGS (SEQ ID NO: 87)

### (5-2) Construction of template DNA for EMP-leucine zipper fusion

As template DNAs for expressing EMP-Jun-cys-N11-SecM-GS3, a nucleotide sequence encoding the above amino acid sequence (i) was designed. Furthermore, a T7 promoter sequence and a ribosome binding sequence necessary for cell-free expression were added to the 5'-side thereof, and a termination codon and an untranslated region were added to the 3'-side thereof to design (ii). In addition, single-stranded DNAs (iii) to (ix) were designed to prepare these template DNAs by PCR.

The single-stranded DNA sequences shown in (iii) to (ix) were totally synthesized at Eurofins Genomics K.K.
(ii) Nucleotide sequence for expressing EMP-Jun-cys-N11-SecM-GS3 
GAAATTAATACGACTCACTATAGGGAGACCACAACGGTTTCCCTCTAGAAATA
ATTTTGTTTAACTTTAAGAAGGAGATATACCAATGGCAGAGACCGGTGGCCTG
TATGCGTGCCATATGGGGCCAATGACCTGGGTGTGTCAGCCGTTACGCGGCG
CAGCTCCCGCAGCACCAGCTCCAGCAGCAGGCGGTCGTATCGCTCGTCTGGA
AGAAAAAGTTAAAACCCTGAAAGCTCAGAACTCCGAACTGGCTTCCACCGC
TAACATGCTGCGTGAACAGGTTGCTCAGCTGAAACAGAAAGTTATGAACGGC
TGCGGTGGCAAAGATCATCTCATCCACAATCATCACAAACATGAGCACGCTC
ATGCCGAACACCTGTTTTCTACTCCTGTTTGGATTTCTCAAGCTCAAGGTATT
 
CGTGCTGGTGGTAGCGGTAGCGGTAGCTAGTGAATAACTAATCC (SEQ ID NO:
88)
(iii) Nucleotide sequence 1 of single-stranded DNA for preparing (ii) (set forth in SEQ ID NO: 77)
(iv) Nucleotide sequence 2 of single-stranded DNA for preparing (ii) 
ATACCAATGGCAGAGACCGGTGGCCTGTATGCGTGCCATATGGGGCCAATGA
CCTGGGTGTGTCAGCCGTTACGCGGCGCAGCTCCCGCAG (SEQ ID NO: 89)
(v) Nucleotide sequence 3 of single-stranded DNA for preparing (ii) 
GGCGCAGCTCCCGCAGCACCAGCTCCAGCAGCAGGCGGTCGTATCGCTCGTC
TGGAAGAAAAAGTTAAAACCCTGAAAGCTCAGAACTCCGAACTGGCTTCCA
CCGCTAACATGCTGCGTGAACAGGTTGCTCAGCTGAAACAGAAAGTTATGAA
C (SEQ ID NO: 90)
(vi) Nucleotide sequence 4 of single-stranded DNA for preparing (ii) (set forth in SEQ ID NO: 82)
(vii) Nucleotide sequence 5 of single-stranded DNA for preparing (ii) (set forth in SEQ ID NO: 83)
(viii) Nucleotide sequence 6 of single-stranded DNA for preparing (ii) (selec_PCR_F1.F39, set forth in SEQ ID NO: 84)
(ix) Nucleotide sequence 7 of single-stranded DNA for preparing (ii) (selec_PCR_R2.R24, set forth in SEQ ID NO: 85)

### (5-3) Preparation of library

The DNA sequence of EMP-Jun-cys-N11-SecM-GS3 was prepared by PCR. Two types of DNA sequences, aMD4dY-Jun-cys-N11-SecM-GS3 and TPO-Jun-cys-N11-SecM-GS3, prepared in Example 4 were used. A DNA mix was prepared by mixing DNAs, EMP-Jun-cys-N11-SecM-GS3, aMD4dY-Jun-cys-N11-SecM-GS3, and TPO-Jun-cys-N11-SecM-GS3, at a ratio of 1:50:50.

### (5-4) Preparation of mRNA library by transcription reaction

DNA transcription was carried out using T7 RNA polymerase. T7 RNA polymerase (Takara Bio, 2540A) and rNTPs (Promega, E6000) as well were used for transcription. 11 µL of ultrapure water, 2 µL of 10 x T7 RNA polymerase buffer, 2 µL of 50 mM DTT, 2 µL of 25 mM rNTPs, 1 µL of T7 RNA polymerase, and 1 µL of DNA mix were mixed and reacted in a constant temperature bath at 37°C for 6 hours. A 50 mM EDTA solution was then added. It was purified with RNA Clean & Concentrator 5 (ZYMO RESEARCH, R1015) and made into a 2 µM mRNAmix.

### (5-5) Translation of mRNA library and formation of ribosome-cyclic peptide-leucine zipper fusion-mRNA complex

PURExpress ΔRF1,2,3 kit was used for translation expression of the prepared library. 2 µL of solution A, 1.5 µL of solution B, 1 µL of mRNA mix, and 0.5 µL of ultrapure water were mixed and cultured in a 37°C constant temperature bath for 2 hours. After the reaction, the reaction vessel was placed on ice, and 20 µL of ice-cold 1 x TBSM-T buffer (50 mM Tris-HCl, 150 mM NaCl, 50 mM MgCl₂, 0.05% Tween 20) was added to the formed ribosome-cyclic peptide-leucine zipper fusion-mRNA complex.

### (5-6) Immobilization of target molecule

Immobilization of target molecules to magnetic beads was carried out. 5 µL of 2 pM Recombinant Human Erythropoietin R Fc Chimera Protein, CF (R&D systems, 963-ER-50), 5 µL slurry of Dynabeads (invitrogen, DB10003), and 10 µL of 1 x TBS-T buffer (50 mM Tris-HCl, 150 mM NaCl, 0.05% Tween 20) were mixed and the mixture was subjected to inversion mixing for 30 minutes at 4°C. The magnetic beads were washed three times with 100 µL of 1 x TBS-T buffer, then resuspended in 10 µL of 1 x TBS-T buffer and allowed to stand on ice.

### (5-7) Mixing and recovery of ribosome-cyclic peptide-leucine zipper fusion-mRNA complex and target molecule

From the magnetic bead suspension, 1 x TBS-T buffer was removed, and the entire amount of the complex reaction solution in (5-5) above was added thereto. After slow resuspension, the mixture was subjected to inversion mixing at 4°C for 30 minutes. The magnetic bead suspension was washed three times with 200 µL of ice-cold 1 x TBSM-T buffer. To magnetic beads isolated using a neodymium magnet, 20 µL of 1 x TBS-T buffer and 5 µL of 500 mM EDTA aqueous solution were added, mixed well, and allowed to stand at room temperature for 5 minutes to release mRNA from the ribosome-cyclic peptide-leucine zipper fusion-mRNA complex.

### (5-8) Purification of mRNA

Using RNA Clean & Concentrator 5, mRNA was purified from the recovered solution.

### (5-9) Reverse transcription of purified mRNA

The purified mRNA was reverse transcribed with SuperScript^{™} II Reverse Transcriptase (Invitrogen, 18064022). 1 µL of the purified mRNA solution, 0.5 µL of 100 µM selec_PCR_R2.R24, 2.5 µL of dNTPs, and 2 µL of ultrapure water were mixed and heated at 70°C for 5 minutes. The mixture was cooled slowly at room temperature and placed in a tabletop centrifuge. 2 µL of 5 x First-strand buffer, 1 µL of 0.1 M DTT solution, 0.5 µL of ultrapure water, and 0.5 µL of SuperScript II Reverse Transcriptase were added in order, which was reacted at 42°C for 50 minutes to obtain an mRNA-cDNA complex.

### (5-10) PCR amplification of cDNA

The recovered cDNA was amplified by PCR. KOD -Plus- Ver. 2 (Toyobo, KOD-211) was used for PCR. To 4 µL of the completed reverse transcription reaction, 10 µL of 10 x PCR buffer, 4 µL of 250 mM MgSO₄, 10 µL of 2 mM dNTPs, 1 µL of 100 µM selec_PCR_F1.F39, 1 µL of 100 µM selec_PCR_R2.R24, 63 µL of ultrapure water, and 2 µL of KOD -Plus- were mixed. A cycle was created under the conditions of 94°C for 30 seconds, 55°C for 40 seconds, and 68°C for 60 seconds, and was repeated 20 to 25 times. The recovered DNA was used as DNA mix2.

### (5-11) DNA length analysis of cDNA

Three types of DNAs, EMP-Jun-cys-N11-SecM-GS3, aMD4dY-Jun-cys-N11-SecM-GS3, and TPO-Jun-cys-N11-SecM-GS3, along with DNA mix and DNA mix2 were evaluated with 2100 Bioanalyzer (Agilent technologies). Fig. 17 shows the results. Lanes 1 to 5 correspond to EMP-Jun-cys-N11-SecM-GS3, aMD4dY-Jun-cys-N11-SecM-GS3, TPO-Jun-cys-N11-SecM-GS3, DNA mix, and DNA mix2 in order, respectively. When the band of DNA mix2 obtained after carrying out the ribosome display method was compared with the band of DNA mix, a shift in the direction of increasing DNA length was confirmed, and in DNA mix2, the band showing the same DNA length as the DNA encoding EMP-Jun-cys-N11-SecM-GS3 showed the highest intensity.

### (5-12) Activity evaluation of cell-free translation product from amplified cDNA

Three types of DNAs, EMP-Jun-cys-N11-SecM-GS3, aMD4dY-Jun-cys-N11-SecM-GS3, and TPO-Jun-cys-N11-SecM-GS3, along with DNA mix and DNA mix2, and further DNA-free distilled water corresponding to the no-addition group were used to carry out cell-free translation with PUREfrex 2.0 (Gene Frontier). To 20 ng of each DNA, 2.5 µL of Solution I, 0.25 µL of Solution II, 0.5 µL of Solution III, and 1.75 µL of water were added, and the mixture was incubated at 37°C for 6 hours to carry out the translation reaction.

These cell-free translation products were evaluated using PathHunter^{®} eXpress EpoR-JAK2 Functional Assay Kit (DiscoveRx). Cells for EPO activity evaluation contained in the PathHunter^{®} eXpress EpoR-JAK2 Functional Assay Kit were seeded in a 96-well plate and cultured for 24 hours at 37°C under 5% CO₂. To the cells, the cell-free expression system reaction solution was added at varying concentrations with a series of five serial dilutions (at concentrations of 1/110, 1/330, 1/990, 1/2970 and 1/8910) in the medium, or 50 µg/mL recombinant human EPO was added at a concentration of 1/110 in the medium. The prepared Substrate Reagent was added to the cells stimulated at room temperature for 3 hours and incubated for 60 minutes. Chemiluminescence intensity was quantified with a Nivo plate reader (Perkin Elmer).

Fig. 18 shows the results. It has been revealed that when DNA mix and DNA mix2 are compared for relative receptor activity calculated from chemiluminescence intensity, only DNA mix2 has activity. Moreover, from the activities of the cell-free translation products of EMP-Jun-cys-N11-SecM-GS3, aMD4dY-Jun-cys-N11-SecM-GS3, and TPO-Jun-cys-N11-SecM-GS3, it has been found that DNA mix2 is enriched EMP-Jun-cys-N11-SecM-GS3.

Based on the above results, it has been revealed that this approach makes it possible to obtain, from a library including a plurality of displayed polypeptides (cyclic peptide-leucine zipper fusions), a cyclic peptide-leucine zipper fusion that has binding affinity with the target molecule and activity based on the binding. Moreover, together with the results of Example 4, it has been shown that the target molecule is not limited either.

## Claims

1. A method for screening dimerized cyclic peptides, including steps of:
(1) synthesizing at least one polypeptide including
a cyclic peptide,
a dimerization domain containing a cysteine residue and a leucine zipper region, and
a peptide linker present between the cyclic peptide and the dimerization domain
in a cell-free expression system to prepare a dimer of the polypeptide; and
(2) measuring activity based on binding of the dimer to a target molecule.

2. A method for screening dimerized cyclic peptides, including steps of:
(1) providing a dimer of at least one polypeptide including
a cyclic peptide,
a dimerization domain containing a cysteine residue and a leucine zipper region, and
a peptide linker present between the cyclic peptide and the dimerization domain,
obtained by synthesizing the polypeptide in a cell-free expression system; and
(2) measuring activity based on binding of the dimer to a target molecule.

3. The method according to claim 1 or 2, wherein in the step (1), the at least one polypeptide includes a plurality of different polypeptides expected to have binding affinity with the target molecule, and the step (2) further includes measuring the activity of dimers of the plurality of different polypeptides based on binding to the target molecule.

4. The method according to any one of claims 1 to 3, wherein the step (1) further includes creating a library including a plurality of the at least one polypeptide or the dimer, or providing a library including a plurality of the at least one polypeptide or the dimer, and
wherein the plurality of polypeptides include different cyclic peptides.

5. A method for creating a library including a plurality of polypeptides or dimers of the plurality of polypeptide, including synthesizing the plurality of polypeptides in a cell-free expression system, wherein
the plurality of polypeptides each include
a cyclic peptide,
a dimerization domain containing a cysteine residue and a leucine zipper region, and
a peptide linker present between the cyclic peptide and the dimerization domain, and
the plurality of polypeptides include different cyclic peptides.

6. A library for screening dimerized cyclic peptides, including:
a plurality of polypeptides or dimers of the plurality of polypeptides; or
a plurality of nucleic acids encoding the plurality of polypeptides, wherein
the plurality of polypeptides each include
a cyclic peptide,
a dimerization domain containing a cysteine residue and a leucine zipper region, and
a peptide linker present between the cyclic peptide and the dimerization domain, and
the plurality of polypeptides include different cyclic peptides.

7. The library according to claim 6, including 8 or more kinds of polypeptides or dimers of the 8 or more kinds of polypeptides, or 8 or more kinds of nucleic acids encoding the 8 or more kinds of polypeptides.

8. A method for screening dimerized cyclic peptides using the library according to claim 6 or 7, including measuring activity based on binding of dimers of a plurality of polypeptides to a target molecule.

9. A method for screening dimerized cyclic peptides, including steps of:
(1) obtaining one or a plurality of polypeptides having binding affinity with a target molecule from a library including a plurality of displayed polypeptides, wherein the plurality of displayed polypeptides each include
a cyclic peptide,
a dimerization domain containing a cysteine residue and a leucine zipper region, and
a peptide linker present between the cyclic peptide and the dimerization domain, and
the plurality of displayed polypeptides include different cyclic peptides,
(2) recovering a nucleic acid associated with the polypeptides obtained in the step (1),
(3) amplifying the nucleic acid recovered in the step (2),
(4) synthesizing a polypeptide including
a cyclic peptide,
a dimerization domain containing a cysteine residue and a leucine zipper region, and
a peptide linker present between the cyclic peptide and the dimerization domain
in a cell-free expression system using the nucleic acid amplified in the step (3) to prepare a dimer of the polypeptide, and
(5) measuring activity based on binding of the dimer to a target molecule.

10. The method according to claim 9, wherein the step (1) includes creating a further library including a plurality of displayed polypeptides based on the obtained plurality of polypeptides, and repeating obtaining from the further library one or more polypeptides having binding affinity with the target molecule.

11. A library for screening dimerized cyclic peptides, including:
a plurality of displayed polypeptides; or
a plurality of nucleic acids for expressing the plurality of displayed polypeptides, wherein
the plurality of displayed polypeptides each include
a cyclic peptide,
a dimerization domain containing a cysteine residue and a leucine zipper region, and
a peptide linker present between the cyclic peptide and the dimerization domain, and
the plurality of displayed polypeptides include different cyclic peptides.

12. The library according to claim 11, including 10⁵ or more kinds of displayed polypeptides or 10⁵ or more kinds of nucleic acids for expressing the 10⁵ or more kinds of displayed polypeptides.

13. A method for obtaining dimerized cyclic peptides having activity based on binding to a target molecule by measuring the activity based on the binding to the target molecule by the method according to any one of claims 1 to 4 and 8 to 10.

14. A method for preparing dimerized cyclic peptides, including:
synthesizing a polypeptide including
a cyclic peptide,
a dimerization domain containing a cysteine residue and a leucine zipper region, and
a peptide linker present between the cyclic peptide and the dimerization domain
in a cell-free expression system to prepare a dimer of the polypeptide.

15. A polypeptide including:
a cyclic peptide,
a dimerization domain containing a cysteine residue and a leucine zipper region, and
a peptide linker present between the cyclic peptide and the dimerization domain.

16. The polypeptide according to claim 15, wherein the dimerization domain has one cysteine residue.

17. The polypeptide according to claim 15 or 16, wherein the peptide linker is composed of one or more amino acid residues selected from alanine (A), proline (P), and serine (S).

18. A nucleic acid encoding the polypeptide according to any one of claims 15 to 17.
